# EUROPÄISCHE PATENTSCHRIFT

(11) **EP 2 091 908 B1**
(45) Veröffentlichungstag und Bekanntmachung des Hinweises auf die Patenterteilung: **13.06.2012**
(21) Anmeldenummer: 07803096.2
(22) Anmeldetag: 31.08.2007
(51) Int. Cl.: C07C 67/56, C07C 69/54, C07C 67/20

(54) **VERFAHREN ZUR ADSORPTIVEN AUFREINIGUNG VON METHACRYLSÄUREALKYLESTERN**
METHOD FOR THE ADSORPTIVE PURIFICATION OF ALKYL METHACRYLATES
PROCÉDÉ PERMETTANT LA PURIFICATION PAR ADSORPTION D'ESTERS ALKYLIQUES DE L'ACIDE MÉTHACRYLIQUE

(30) Priorität: 18.12.2006 DE 102006060161
(43) Veröffentlichungstag der Anmeldung: 26.08.2009
(73) Patentinhaber: Evonik Röhm GmbH, 64293 Darmstadt (DE)
(72) Erfinder: GROPP, Udo, 83093 Bad Endorf (DE); WEBER, Robert, 64665 Alsbach-Hähnlein (DE); SCHÄFER, Thomas, 64572 Büttelborn (DE); PERL, Andreas, 67240 Bobenheim-Roxheim (DE); SING, Rudolf, 67551 Worms (DE); MERTZ, Thomas, 64625 Bensheim (DE)
(86) Internationale Anmeldenummer: PCT/EP2007/059090
(87) Internationale Veröffentlichungsnummer: WO 2008/074522

(56) Entgegenhaltungen:
- EP-A- 0 686 623
- WO-A-79/00251
- GB-A- 1 222 993
- JP-A- 8 310 979
- JP-A- 2006 028 080
- ROBERT V. SLONE: "Methacrylic Ester Polymers" ENCYCLOPEDIA OF POLYMER SCIENCE AND TECHNOLOGY, Bd. 3, 22. Oktober 2001 (2001-10-22), Seiten 249-277, XP002463013

## Beschreibung

Die Erfindung betrifft im Allgemeinen ein Verfahren zur Aufreinigung eines Methacrylsäurealkylesters, ein Verfahren zur Herstellung eines Reinst Methacrylsäurealkylesters, eine Vorrichtung zur Herstellung von Methacrylsäurealkylestern, ein Verfahren zur Herstellung eines zumindest teilweise auf Methacrlysäurealkylestern basierenden Polymers, Verwendung eines erfindungsgemäße erhaltenen Methacrylsäurealkylesters in chemischen Produkten sowie einen erfindungsgemäß erhaltene Methacrylsäurealkylester beinhaltende chemische Produkte.

Der Bedarf an hochreinen Methacrylsäurealkylestern steigt stetig. Derartige Ester bilden die Basis von in der Medizintechnik eingesetzten polymeren Formkörpern, an die höchste Anforderungen in Bezug auf ihre toxische Unbedenklichkeit gestellt werden. Aus dem Stand der Technik sind eine Vielzahl von Aufreinigungsverfahren bekannt, wobei die destillative Aufreinigung am weitesten verbreitet ist. Obgleich sich die destillative Aufarbeitung sehr gut auch in einem kontinuierlich betriebenen Prozess einsetzen lässt, ist es sehr schwierig und mit einem hohen technischen Aufwand verbunden, durch Destillation Methacrylsäurealkyester in sehr hohen Reinheiten bereitzustellen. Diese Schwierigkeiten und der hohe Aufwand sind vor allen Dingen in der geringen thermischen Belastbarkeit und der spontanen Polymerisationsneigung der durchaus reaktiven Methacrylsäurealkylester begründet. Insbesondere durch die Polymerisationsneigung der Methacrylsäurealkylester ist es immer wieder notwendig, den Betrieb von Destillationskolonnen in großtechnischen Anlagen zu unterbrechen, um diese mit aufwändigen und zeitraubenden Verfahren von den sich in den Kolonnen und Kolonneneinbauten gebildeten Polymer zu befreien.

JP 2006028080 offenbart die Verwendung von Kieselgel als Adsorbens zur Reinigung von Methacrylsäureestern. Die Reinigung durch Adsorption erfolgt dabei nach der Destillation des Rohmethacrylsäureesters. Die Methacrylsäureester können durch Umesterung von Methacrylsäureestern mit entsprechenden Alkoholen erhalten werden bzw. durch Veresterung von Methacrylsäure. Mit diesem Verfahren können Metallverunreinigungen, der Polymerisationsinhibitor und Oligomere durch Absorption an einem Reinigungsstoff entfernt werden.

JP 08310979 offenbart Kieselgel, Molekularsieb und Zeolithe als Adsorptionsmittel zur Reinigung von Acryl- und Methacrylsäureestern. Es wird die Entfernung von Polymerisationsinhibitoren beschrieben.

GB 1222993 offenbart, dass Acrylsäure- oder Methacrylsäureester gereinigt werden können, indem sie über eine Schicht Molekularsieb filtriert werden. Es wird ein spezielles und aufwendiges Destillationsverfahren offenbart, welches jedoch keine Entfernung von Ameisensäure und/oder Methacrylsäure und/oder Schwefelsäure beschreibt.

JP 63002952 offenbart ein Verfahren zur Reinigung von Methylmethacrylat beinhaltend die Behandlung mit einem schwach basischen Ionenaustauscherharz, wobei organische Säuren wie Methacrylsäure entfernt werden. Es wird keine Entfernung von Ameisensäure und/oder Methacrylsäure und/oder Schwefelsäure beschrieben.

Vorliegend besteht die erfindungsgemäße Aufgabe im Allgemeinen darin, die sich aus dem Stand der Technik ergebenden Nachteile zumindest teilweise zu überwinden.

Insbesondere liegt eine erfindungsgemäße Aufgabe darin, ein Verfahren und eine Vorrichtung bereitzustellen, mit der Methacrylsäurealkylestern in höchster Reinheit einem im Vergleich zum Stand der Technik geringeren Aufwand und insbesondere möglichst ohne Bildung von unerwünschten Polymeren erfolgt.

Weiterhin besteht eine erfindungsgemäße Aufgabe darin, auf Methacrylsäurealkylestern basierende Polymere bereitzustellen, die toxikologisch besonders unbedenklich sind.

So lag ferner eine Aufgabe der vorliegenden Erfindung darin, die unerwünschte Polymerisation der zu reinigenden Methacrylsäurealkylester möglichst weitgehend zu vermeiden.

Außerdem lag eine erfindungsgemäße Aufgabe darin, ein Reinigungsverfahren bereit zustellen, dass sich möglichst wenig nachteilig auf die Weiterverarbeitunkschritte des erfindungsgemäß aufgereinigten Methacrylsäurealkylesters und die so erhaltenen Weiterverarbeitungsprodukte ausübt.

Zudem lag eine erfindungsgemäße Aufgabe darin, ein Reinigungsverfahren bereitzustellen, das einen geringen Energieverbrauch hat und gleichwohl zu befriedigenden Reinheiten des zu reinigenden Methacrylsäurealkylesters führt.

Einen Beitrag zur Lösung der vorstehenden Aufgaben ergibt sich aus dem Gegenstand der kategoriebildenden Ansprüche, wobei die davon abhängigen Unteransprüche bevorzugte Ausgestaltungen der vorliegenden Erfindung darstellen.

So betrifft die Erfindung ein Verfahren zur Aufreinigung eines Methacrylsäureesters, beinhaltend die Schritte:
i. Bereitstellen eines Methacrylsäurealkylesters mit einer Verunreinigung;
ii. Absorption mindestens eines Teils der Verunreinigung an ein Reinigungsfeststoff und Erhalt eines Reinst Methacrylsäurealkylesters.

Im Zusammenhang mit dem Reinigungsfeststoff ist es erfindungsgemäß bevorzugt, dass dieser aus einer Vielzahl von Reinigungsfeststoffteilchen besteht, die in einem Reinigungsbehälter aufgenommen sind. In dem der Reinigungsbehälter durch den flüssigen Methacrylsäureester mit einer Verunreinigung durchströmt wird, werden die Vielzahl von Teilchen des Reinigungsstoffs mit der flüssigen Mischung aus Methacrylsäurealkylester und der mindestens einen Verunreinigung umströmt. Hierbei ist es vorteilhaft, dass die Durchströmung des Reinigungsbehälters und die Umströmung der Reinigungsfeststoffteilchen möglichst gleichmäßig erfolgt. In diesem Zusammenhang hat es sich bewährt, dass die Durchströmungsgeschwindigkeit des Reinigungsbehälters in einem Bereich von etwa 2 bis etwa 30, vorzugsweise in einem Bereich von etwa 2 bis etwa 10 und besonders bevorzugt in einem Bereich von etwa 3 bis etwa 9 m/s liegt.

Ferner ist es erfindungsgemäß bevorzugt, dass der Reinigungsfeststoff eine spezifische Oberfläche in einem Bereich von etwa 400 bis etwa 1500 m²/g, vorzugsweise in einem Bereich von etwa 500 bis etwa 1000 m²/g und besonders bevorzugt in einem Bereich von etwa 650 bis etwa 800 m²/g aufweist. Weiterhin ist es erfindungsgemäß bevorzugt, dass der Reinigungsfeststoff Poren aufweist, die vorzugsweise so dimensioniert sind, dass die Verunreinigungen daran gut adsorbiert werden können. Daher kann es durchaus möglich sein, dass der Reinigungsfeststoff unterschiedliche Porengrößen aufweist. Dieses kann beispielsweise dadurch erreicht werden, in dem drei oder mehr verschiedene Reinigungsfeststoffe unterschiedliche Porengröße miteinander gemischt werden. Im Allgemeinen hat sich ein nach DIN 66135-1 volumetrisch bestimmte Porendurchmesser des Reinigungsfeststoffs in einem Bereich von etwa 0,1 bis etwa 2 nm, vorzugsweise in einem Bereich von etwa 0,2 bis etwa 0,8 nm und besonders bevorzugt in einem Bereich von etwa 0,3 bis etwa 0,6 nm bewährt.

Neben der Schüttdichte, die bei den Reinigungsfeststoffen vorzugsweise in einem Bereich von etwa 800 bis etwa 1100 kg/m³, aber auch oft in einem Bereich von etwa 920 bis 960 kg/ m³ liegt, beeinflussen die Teilchengröße und die Teilchengrößenverteilung der Reinigungsfeststoffteilchen die Durchströmbarkeit des diese beinhaltenden Reinigungsbehälters der zu reinigenden Mischung aus Methacrylsäurealkylester und mindestens einer Verunreinigung. Hierzu ist es erfindungsgemäß bevorzugt, dass mindestens 80 Gew.-% der Teilchen des Reinigungsstoffs eine Teilchengröße in einem Bereich von etwa 0,5 bis etwa 10 mm, vorzugsweise in einem Bereich von etwa 1 bis etwa 8 mm und besonders bevorzugt in einem Bereich von etwa 1,2 bis etwa 7,5 mm aufweisen. Ferner ist es bevorzugt, dass das Gewichtsmittel der Teilchengröße des Reinigungsfeststoffs in einem Bereich von etwa 0,5 bis etwa 10 mm, vorzugsweise in einem Bereich von etwa 1 bis etwa 8 mm und besonders bevorzugt in einem Bereich von etwa 1,2 bis 7,5 mm liegt. In manchen Fällen liegt mindestens ein Teil des Reinigungsstoffs in zylinderartigen Einheiten wie Stranggranulat vor. Hierbei ist es bevorzugt, dass diese Einheiten einen Querschnitt in einem Bereich von etwa 0,5 bis etwa 5 mm, vorzugsweise in einem Bereich von etwa 1 bis etwa 4 mm und besonders bevorzugt in einem Bereich von etwa 1,4 bis 2 mm sowie eine Länge in einem Bereich von etwa 3 bis etwa 12 mm, vorzugsweise in einem Bereich von etwa 4 bis etwa 10 mm und besonders bevorzugt in einem Bereich von etwa 5 bis 9 mm aufweisen.

Weitere Maßnahmen im Zusammenhang mit der Verbesserung der Durchströmung des Reinigungsbehälters und damit des Reinigungsfeststoffs kann durch Einbauten und Packungen in dem Reinigungsbehälter erzielt werden. So kann der Reinigungsbehälter einen Einlass mit einem kleineren Durchmesser zur Aufnahme der zu reinigenden Flüssigkeit als der Bereich des Reinigungsbehälters, der den Reinigungsfeststoff aufnimmt. Vorzugsweise ist der Reinigungsbehälter stumpfkegelförmig ausgestaltet. Hierbei kann der Deckflächenbereich den Einlass und die Grundfläche den Auslass bilden. Auf den Einlass folgend kann der Reinigungsbehälter eine Reihe von dem Reinigungsfeststoff verschiedene Packungen aufweisen, die dazu dienen, den Reinigungsfeststoff zu halten und einem zu leichten Ausschlämmen des Reinigungsfeststoffes zu begegnen. So können diese Packungen, in Durchströmungsrichtung, betrachtet unter und über dem Reinigungsfeststoff vorgesehen sein.

Außerdem entspricht es einer erfindungsgemäßen Ausgestaltung, dass der Reinigungsfeststoff nicht als eine Vielzahl von Teilchen sondern als ein Formkörper oder in einer Kombination von zwei und mehr Formkörpern vorliegt, die von einer Vielzahl von Kanälen durchzogen sind, die durch die Mischung aus Methacrylsäurealkylester und mindestens einer Verunreinigung durchströmt werden. Durch Wahl der Ausgestaltung dieser Kanäle, insbesondere deren Länge und Durchmesser, kann die Durchströmungsgeschwindigkeit und damit die Verweilzeit der Mischung aus Methacrylsäurealkylester und mindestens einer Verunreinigung so gewählt werden, dass einerseits ausreichend Zeit zur Adsorption eines möglichst großen Teils der Verunreinigung an dem Reinigungsfeststoff und andererseits eine wirtschaftlich sinnvolle Verweilzeit eingestellt werden kann. Die Verweilzeit der Methacrylsäurealkylesters in dem Behälter kann in einem Bereich von etwa 0,15 bis etwa 20 h, vorzugsweise in einem Bereich von etwa 0,3 bis etwa 10 h und besonders bevorzugt in einem Bereich von etwa 0,4 bis etwa 2 h.

Grundsätzlich kommt als Reinigungsfeststoff jedes dem Fachmann bekanntes und geeignet erscheinendes Material das in der Lage ist mindestens einen Teil der Verunreinigungen in der Mischung aus Methacrylsäurealkylester und mindestens einer Verunreinigung zu adsorbieren. In diesem Zusammenhang ist es erfindungsgemäß dass der Reinigungsfeststoff mindestens 10 Gew.-%, vorzugsweise mindestens 50 Gew.-% und besonders bevorzugt mindestens 75 Gew.-%, jeweils bezogen auf den Reinigungsfeststoff, eine Silizium-Sauerstoff-Verbindung, beinhaltet. Hierunter fallen vor allen Dingen die Oxyde des Siliziums (vgl. Holleman-Wiberg *"Lehrbuch der Anorganischen Chemie"* 91.-100. Auflage, 1985, S. 750 ff.). Erfindungsgemäß ist es besonders bevorzugt, dass der Reinigungsstoff mindestens 10 Gew.-%, vorzugsweise mindestens 50 Gew.-% und besonders bevorzugt mindestens 75 Gew.-% eines Zeoliths, vorzugsweise vom Typ A, beinhaltet. Insbesondere im Fall von anorganischen Reinigungsfeststoffen ist es möglich, diese thermisch zu regenerieren. Hierzu kann der Reinigungsfeststoff über einen gewissen Zeitraum, meist in einem Bereich von etwa 10 Minuten und 30 h, vorzugsweise in eine Bereich von etwa 2 bis 28 h und besonders bevorzugt in einem Bereich von etwa 3 bis etwa 24 h, bei einer Temperatur in einem Bereich von etwa 300 bis etwa 1.800°C, vorzugsweise in einem Bereich von etwa 700 bis etwa 1.300°C geglüht, so dass die meist organischen Verunreinigungen in Kohlendioxid überführt werden und so der Reinigungsfeststoff von der Verunreinigungsfracht befreit wird. Das erfindungsgemäße Verfahren kann weitere Schritte enthalten. Hierbei ist es bevorzugt, dass als zusätzlicher Schritt eine thermische Generierung des Reinigungsfeststoffs, gefolgt von der Wiederverwertung des regenerierten Reinigungsfeststoffs in Schritt ii. erfolgt. Weiterhin ist es erfindungsgemäß bevorzugt, dass mindestens der Schritt ii. kontinuierlich erfolgt. Hierbei wird unter kontinuierlich verstanden, dass ein dauerhafter Betrieb erfolgt, der lediglich durch Wartungs- und Reinigungsarbeiten unterbrochen wird.

Weiterhin kann es erfindungsgemäß bevorzugt sein, dass verschiedene Reinigungsverfahren miteinander kombiniert werden. So kann der Methacrylsäurealkylester vor dem erfindungsgemäßen Schritt ii. zur Vorreinigung destilliert werden.

Als Verunreinigungen kommen im allgemeinen alle im Rahmen der Herstellung von Methacrylsäurealkylester anfallenden Verunreinigungen in Betracht. Erfindungsgemäss kommen Ameisensäure, Methacrylsäure oder Schwefelsäure oder mindestens zwei davon vor Weiterhin ist es erfindungsgemäß bevorzugt, dass der in Schritt i. bereitgestellte Methacrylsäurealkylester mindestens eine, oder mindestens zwei, oder jede der nachfolgenden Verunreinigungen beinhaltet:
a. Ameisensäure in einer Menge von maximal 0,05 Gew.-%, vorzugsweise maximal 0,01 Gew.-% und besonders bevorzugt maximal 0,005 Gew.-%;
b. Methacrylsäure in einer Menge von maximal 0,1 Gew.-%, vorzugsweise maximal 0,02 Gew.-% und besonders bevorzugt maximal 0,01 Gew.-%;
c. Schwefeldioxid in einer Menge von maximal 0,001 Gew.-%, vorzugsweise maximal 0,0005 Gew.-% und besonders bevorzugt maximal 0,0001 Gew.-%.

Ferner ist es erfindungsgemäß bevorzugt, dass die durch das erfindungsgemäße Verfahren mindestens ein Stoff und vorzugsweise mindestens zwei Stoffe, die die Verunreinigungen der in Schritt i. bereitgestellten Methacrylsäurealkylester darstellen, um mindestens 5, vorzugsweise mindestens 30 und besonders bevorzugt mindestens 60 % dem Reinst-Methacrylsäurealkylester abgereichert sind.

Durch den Reinigungsfeststoff lassen sich die Verunreinigungen nahezu vollständig abreichern. So dass die durch den Reinigungsfeststoff aufgereinigte Methacrylsäurealkylester mit noch nicht über den Reinigungsfeststoff geführten Methacrylsäurealkylester gemischt werden kann, um beispielsweise ein spezifikationsgerechte Reinheit zu erhalten. Daher ist es möglich, dass die Methacrylsäurealkylester-Ströme nur teilweise als Bypass über den Reinigungsfeststoff zu führen.

Zudem betrifft die Erfindung ein Verfahren zur Herstellung eines Reinst-Methacrylsäurealkylesters, beinhaltend als Schritte
- Erzeugen eines gegebenenfalls vorgereinigten Methacrylsäurealkylesters;
- Reinigen des Methacrylsäurealkylesters durch das erfindungsgemäße Verfahren zur Aufreinigung eines Methacrylsäurealkylesters.

Es ist weiterhin bevorzugt, dass das erfindungsgemäße Verfahren zur Herstellung eines Reinst-Methacryläsureesters die folgenden Schritte zusätzlich beinhaltet:
i. Bereitstellen eines Acetoncyanhydrins;
ii. In Kontakt bringen des Acetoncyanhydrins mit einer anorganischen Säure unter Erhalt eines Methacrylamids;
iii. In Kontakt bringen des Methacrylamids mit einem Alkohol unter Erhalt eines Methacrylsäureesters;
iv. gegebenenfalls Vorreinigen des Methacrylsäurealkylesters.

Außerdem betrifft die vorliegende Erfindung auch eine Vorrichtung zur Herstellung von Methacrylsäurealkylestern, beinhaltend fluidleitend miteinander verbunden:
- ein Anlagenelement zur Herstellung von Acetoncyanhydrin, gefolgt von;
- einem Anlagenelement zur Herstellung von Methacrylamid, gefolgt von;
- einem Anlagenelement zur Herstellung von Methacrylsäurealkylester, gegebenenfalls gefolgt von;
- einem Anlagenelement zur Reinigung des Methacrylsäureesters, gegebenenfalls gefolgt von;
- einem Anlageelement zur Polymerisation, gegebenenfalls gefolgt von;
- einem Anlagenelement zur Konfektionierung, wobei das Anlagenelement zur Reinigung einen Behälter mit einem Reinigungsfeststoff enthaltend mindestens 10% einer Silicium-Sauerstoff- Verbindung aufweist.

Unter fluidleitend verbunden wird vorliegende verstanden, dass Gase, Flüssigkeiten und Gas-Flüssigkeiten-Gemische oder andere fließfähige Stoffe geleitet werden können. Im Zusammenfang mit den einzelnen Ausgestaltungen des Reinigungsfeststoffs wird auf die vorhergehenden Ausführungen Bezug genommen.

Weiterhin ist es erfindungsgemäß bevorzugt, dass das erfindungsgemäße Verfahren der vorstehend beschriebenen erfindungsgemäßen Vorrichtung erfolgt.

Zudem betrifft die Erfindung ein Verfahren zur Herstellung von zumindest teilweise auf Methacrylsäurealkylestern basierenden Polymeren, beinhaltend die Schritte:
A. Bereitstellen eines Reinst-Methacrylsäurealkylesters nach dem vorstehend beschriebenen erfindungsgemäßen Verfahren;
B. Polymerisieren, vorzugsweise radikalisch, des Reinst-Methacrylsäurealkylester und gegebenenfalls mindestens einem Comonomer unter Erhalt eines Polymerisats;
C. Aufarbeiten des Polymerisats unter Erhalt eines Polymers.

Als Comonomere kommen sämtliche dem Fachmann bekannten und geeignet erscheinenden in Betracht, wobei radikalisch polymerisierbare Monomere besonders bevorzugt sind. Unter diesen sind vor allem Styrol, Butylacrylat oder Acrylnitril zu nennen. Polymerisation kann als Lösungs-, Perl-, Emulsions-, oder Suspensionspolymerisation, auch als Substanzpolymerisation durchgeführt werden. Die Aufarbeitung des Polymerisats erfolgt beispielsweise durch Fällen des Lösemittel beinhaltenden Polymerisats in einem Nichtlösemittel für das Polymer als Fällungsmittel. So wird beispielsweise ein Aceton als Lösungsmittel und Polymethylmethacrylat aufweisendes Polymerisat in einem Fällungsmittel aus Methanol und Wasser gefällt, vom Fällungsmittel getrennt und getrocknet.

Zudem betrifft die Erfindung die Verwendung eines durch das erfindungsgemäße Verfahren erhältlichen Reinst-Methacrylsäurealkylester in Fasern, Filmen, Lacken, Formmassen, Formkörpern, Papierhilfsmittel, Lederhilfsmittel, Flockungsmittel und Bodenzusätze als bevorzugte chemische Produkte.

Zudem betrifft die Erfindung Fasern, Filme, Lacke, Formmassen, Formkörper, Papierhilfsmittel, Lederhilfsmittel, Flockungsmittel und Bohrzusätze als bevorzugte chemische Produkte, die auf einem nach dem erfindungsgemäßen Verfahren erhältlichen Reinst-Methacrylsäureester basieren.

Nachfolgend werden verschiedene Verfahrenselemente und Anlagenteile erläutert, die grundsätzlich einzeln oder als Ensemble aus zwei oder mehr der genannten Verfahrenselemente mit der vorliegenden Erfindung verbindbar sind. Gegebenenfalls kann es vorteilhaft sein, wenn die im Rahmen des vorliegenden Textes vorgestellten Verfahrenselemente derart mit der vorliegenden Erfindung kombiniert werden, dass sie insgesamt zu einem Verfahren zur Herstellung von Estern der Methacrylsäure oder einem Verfahren zur Herstellung von Methacrylsäure kombiniert werden. Es sei jedoch auch darauf hingewiesen, dass sich zumeist vorteilhafte Effekte auch dann erzielen lassen, wenn der Gegenstand der vorliegenden Erfindung als solcher in einem anderen Umfeld eingesetzt wird oder nur mit einem Teil der hier vorgestellten Verfahrenselemente kombiniert wird.

### Herstellung von Acetoncyanhydrin

In diesem Verfahrenselement wird Acetoncyanhydrin nach allgemein bekannten Verfahren (siehe beispielsweise Ullmanns Enzyklopädie der technischen Chemie, 4. Auflage, Band 7) hergestellt. Häufig werden dabei als Reaktionspartner Aceton und Blausäure eingesetzt. Bei der Umsetzung handelt es sich um eine exotherme Reaktion. Um einer Zersetzung des im Rahmen dieser Reaktion gebildeten Acetoncyanhydrins entgegenzuwirken, wird üblicherweise die Reaktionswärme durch eine geeignete Vorrichtung abgeführt. Die Umsetzung kann dabei grundsätzlich als Batchprozess oder als kontinuierliches Verfahren geführt werden, sofern eine kontinuierliche Fahrweise bevorzugt ist, wird die Umsetzung häufig in einem Schlaufenreaktor, der entsprechend eingerichtet ist, durchgeführt.

Ein Hauptmerkmal einer in hohen Ausbeuten zum gewünschten Produkt führenden Fahrweise liegt oft darin, dass bei ausreichender Reaktionszeit das Reaktionsprodukt gekühlt wird und das Reaktionsgleichgewicht in Richtung des Reaktionsproduktes verschoben wird. Darüber hinaus wird das Reaktionsprodukt zum Vorteil der Gesamtausbeute häufig mit einem entsprechenden Stabilisator versetzt, um einer Zersetzung bei der späteren Aufarbeitung in die Ausgangsstoffe vorzubeugen.

Die Vermischung der Reaktionspartner Aceton und Blausäure kann grundsätzlich auf im Wesentlichen beliebige Weise erfolgen. Die Art der Vermischung hängt insbesondere davon ab, ob eine diskrete Fahrweise, beispielsweise im Batchreaktor, oder eine kontinuierliche Fahrweise, beispielsweise im Schlaufenreaktor, gewählt wird.

Grundsätzlich kann es vorteilhaft sein, wenn das Aceton in die Reaktion über einen Vorlagebehälter eingespeist wird, der über einen Waschturm verfügt. Entlüftungsleitungen, die Aceton und Blausäure enthaltende Abluft führen, können so beispielsweise durch diesen Vorlagebehälter geführt werden. Im Waschturm, der an den Vorlagebehälter angeschlossen ist, kann die aus dem Vorlagebehälter entweichende Abluft mit Aceton gewaschen werden, wodurch Blausäure aus der Abluft entfernt und in den Prozess rückgeführt werden. Hierzu wird beispielsweise ein Teil der vom Vorlagebehälter in die Reaktion eingeführten Acetonmenge im Teilstrom über einen Kühler, vorzugsweise über einen Sole-Kühler, in den Kopf des Waschturms geführt und so das gewünschte Ergebnis erzielt.

Je nach Umfang der zu produzierenden Menge an Endprodukten kann es vorteilhaft sein, dass Aceton aus mehr als nur einem Vorlagebehälter der Reaktion zuzuführen. Dabei kann jeder der zwei oder mehr Vorlagebehälter einen entsprechenden Waschturm tragen. Es ist jedoch in vielen Fällen ausreichen, wenn nur einer der Vorlagebehälter mit einem entsprechenden Waschturm ausgerüstet ist. In diesem Fall ist es jedoch oft sinnvoll, wenn entsprechende Abluft führende Leitungen, die Aceton und Blausäure transportieren können, über diesen Behälter bzw. über diesen Waschturm geführt werden.

Die Temperatur des Acetons im Vorlagebehälter kann grundsätzlich innerhalb eines im Wesentlichen beliebigen Bereiches liegen, insofern das Aceton sich bei der entsprechenden Temperatur im flüssigen Zustand befindet. Vorteilhafterweise beträgt die Temperatur im Vorlagebehälter jedoch etwa 0 bis etwa 20 ° C.

Im Waschturm wird das zum Waschen eingesetzte Aceton über einen entsprechenden Kühler, beispielsweise über einen Plattenkühler mit Sole auf eine Temperatur von etwa 0 bis etwa 10° C gekühlt. Die Temperatur des Acetons beim Eintritt in den Waschturm beträgt daher vorzugsweise beispielsweise etwa 2 bis etwa 6 ° C.

Die im Rahmen der Reaktion benötigte Blausäure kann entweder in flüssiger oder in gasförmiger Form in den Reaktor eingeführt werden. Es kann sich dabei beispielsweise um Rohgas aus dem BMA- oder aus dem Andrussow-Prozess handeln.

Der Cyanwasserstoff kann beispielsweise verflüssigt werden, beispielsweise durch den Einsatz einer entsprechenden Kühlsole. Anstelle von verflüssigter Blausäure kann Kokereigas eingesetzt werden. So werden beispielsweise Cyanwasserstoff enthaltende Kokereigase nach einer Wäsche mit Pottasche kontinuierlich im Gegenstrom mit Aceton, das 10 % Wasser enthält, gewaschen und die Reaktion zu Acetoncyanhydrin kann in Gegenwart eines basischen Katalysators in zwei hintereinander geschalteten Gaswaschkolonnen durchgeführt werden.

Im Rahmen einer weiteren Ausführungsform kann ein Cyanwasserstoff und inerte Gase enthaltendes Gasgemisch, insbesondere ein Rohgas aus dem BMA- oder aus dem Andrussow-Prozess mit Aceton in Gegenwart eines basischen Katalysators und Acetoncyanhydrin in einem Gas-Flüssig-Reaktor umgesetzt werden.

Im Rahmen des hier beschriebenen Verfahrens wird vorzugsweise ein BMA-Rohgas oder ein Andrussow-Rohgas eingesetzt. Dass aus den oben genannten üblichen Verfahren zur Herstellung von Cyanwasserstoff resultierende Gasgemisch kann als solches oder nach einer Säurewäsche verwendet werden. Das Rohgas aus dem BMA-Prozess, bei welchem aus Methan und Ammoniak im Wesentlichen Blausäure und Wasserstoff gebildet werden, enthält typischerweise 22,9 Vol.-% HCN, 71,8 Vol.-% H₂, 2,5 Vol.-% NH3, 1,1 Vol.-% N₂, 1,7 Vol.-% CH₄. Im bekannten Andrussow-Prozess werden aus Methan und Ammoniak und Luftsauerstoff Blausäure und Wasser gebildet. Das Rohgas des Andrussow-Prozesses enthält beim Einsatz von Sauerstoff als Sauerstoffquelle typischerweise 8 Vol.-% HCN, 22 Vol.% H₂O, 46,5 Vol.-% N₂, 15 Vol.-% H₂, 5 Vol.-% CO, 2,5 Vol.-% NH3 und je 0,5 Vol.-% CH4 und CO₂.

Beim Einsatz eines nicht säuregewaschenen Rohgases aus dem BMA-oder Andrussow-Prozess wirkt der im Rohgas enthaltene Ammoniak häufig als Katalysator für die Reaktion. Da der im Rohgas enthaltene Ammoniak häufig die als Katalysator erforderliche Menge übersteigt und deshalb zu hohen Verlusten an zur Stabilisierung eingesetzter Schwefelsäure führen kann, wird ein derartiges Rohgas oft einer Säurewäsche unterzogen, um Ammoniak daraus zu eliminieren. Beim Einsatz eines solchen Säure gewaschenen Rohgases muss dann allerdings ein geeigneter basischer Katalysator dem Reaktor in katalytischer Menge zugesetzt werden. Im Prinzip können dabei bekannte anorganische oder organische basische Verbindungen als Katalysator fungieren.

Cyanwasserstoff in gasförmiger oder in flüssiger Form bzw. ein Cyanwasserstoff enthaltendes Gasgemisch und Aceton werden im Rahmen der kontinuierlichen Fahrweise fortlaufend einem Schlaufenreaktor zugeführt. Der Schlaufenreaktor umfasst dabei mindestens eine Möglichkeit zum Zuführen von Aceton oder zwei oder mehr solcher Möglichkeiten, mindestens eine Möglichkeit zur Zuführung von flüssiger oder gasförmiger Blausäure, oder zwei oder mehr solcher Möglichkeiten sowie mindestens eine Möglichkeit zum Zuführen eines Katalysators.

Als Katalysator eignen sich grundsätzlich beliebige alkalische Verbindungen wie Ammoniak, Natronlauge oder Kalilauge, die die Umsetzung von Aceton und Blausäure zu Acetoncyanhydrin katalysieren können. Es hat sich jedoch als vorteilhaft herausgestellt, wenn als Katalysator ein organischer Katalysator, insbesondere ein Amin eingesetzt wird. Geeignet sind beispielsweise sekundäre oder tertiäre Amine, wie Diethylamin, Dipropylamin, Triethylamin, Tri-n-propylamin und dergleichen.

Ein im Rahmen des beschriebenen Verfahrenselementes einsetzbarer Schlaufenreaktor weist darüber hinaus noch mindestens eine Pumpe, oder zwei oder mehr Pumpen, und mindestens eine Mischvorrichtung, oder zwei oder mehr solcher Mischvorrichtungen auf.

Als Pumpe eignen sich grundsätzlich alle Pumpen, die geeignet sind, den Umlauf des Reaktionsgemisches im Schlaufenreaktor zu gewährleisten.

Als Mischvorrichtungen sind sowohl Mischvorrichtungen mit beweglichen Elementen als auch so genannte Statikmischer geeignet, bei denen unbewegliche Strömungswiderstände vorgesehen sind. Im Falle des Einsatzes von Statikmischern sind beispielsweise solche geeignet, die einen Betriebsübertrug von mindestens etwa 10, beispielsweise mindestens etwa 15 oder mindestens etwa 20 bar unter Betriebsbedingungen ohne wesentliche Einschränkungen der Funktionsfähigkeit erlauben. Entsprechende Mischer können aus Kunststoff oder Metall bestehen. Als Kunststoff eignen sich beispielsweise PVC, PP; HDPE, PVDF, PFA oder PTFE. Metallmischer können beispielsweise aus Nickellegierungen, Zirkonium, Titan und dergleichen bestehen. Ebenfalls geeignet sind beispielsweise Rechteckmischer.

Die Zugabe des Katalysators erfolgt vorzugsweise im Schlaufenreaktor nach der Pumpe und vor einem im Schlaufenreaktor vorhandenen Mischelement. Katalysatoren werden im Rahmen der beschriebenen Reaktion beispielsweise in einer solchen Menge eingesetzt, dass die Gesamtreaktion bei einem pH-Wert von maximal 8, insbesondere maximal etwa 7,5 oder etwa 7 gefahren wird. Es kann bevorzugt sein, wenn der pH-Wert bei der Reaktion sich innerhalb eines Rahmens von etwa 6, 5 bis etwa 7, 5, beispielsweise etwa 6,8 bis etwa 7,2 bewegt.

Es ist im Rahmen des beschriebenen Verfahrens alternativ zur Zugabe des Katalysators in den Schlaufenreaktor nach der Pumpe und vor einer Mischvorrichtung auch möglich, den Katalysator zusammen mit dem Aceton in den Schlaufenreaktor einzuspeisen. In einem solchen Fall kann es vorteilhaft sein, wenn für eine entsprechende Vermischung von Aceton und Katalysator vor Einspeisung in den Schlaufenreaktor gesorgt wird. Eine entsprechende Vermischung kann beispielsweise durch den Einsatz eines Mischers mit beweglichen Teilen oder durch Einsatz eines Statikmischers erfolgen.

Wenn im Rahmen des beschriebenen Verfahrens eine kontinuierliche Fahrweise in einem Schlaufenreaktor als Betriebsart gewählt wird, so kann es zweckmäßig sein, den Zustand des Reaktionsgemisches durch punktuelle oder fortlaufende Analysen zu untersuchen. Dies bietet den Vorteil, dass gegebenenfalls auch auf Zustandsänderungen im Reaktionsgemisch schnell reagiert werden kann. Darüber hinaus lassen sich so beispielsweise die Reaktionspartner möglichst genau dosieren, um Ausbeuteverluste zu minimieren.

Eine entsprechende Analytik kann beispielsweise durch Probennahme in der Reaktorschlaufe erfolgen. Geeignete Analyseverfahren sind beispielsweise pH-Messung, Messung der Wärmetönung oder Messung der Zusammensetzung des Reaktionsgemisches durch geeignete spektroskopische Verfahren.

Insbesondere im Rahmen der Umsatzkontrolle, Qualitätsaspekten und Sicherheit hat es sich häufig bewährt, den Umsatz im Reaktionsgemisch über die aus dem Reaktionsgemisch abgeführte Wärme zu bestimmen und mit der theoretisch frei werdenden Wärme zu vergleichen.

Die eigentliche Reaktion kann bei geeigneter Wahl des Schlaufenreaktors grundsätzlich in den innerhalb des Schlaufenreaktors angeordneten Rohrsystemen erfolgen. Da die Reaktion jedoch exotherm ist, sollte, um Ausbeuteverlust zu vermeiden, auf eine ausreichende Kühlung bzw. auf eine ausreichende Abfuhr der Reaktionswärme geachtet werden. Es hat sich häufig als vorteilhaft erwiesen, wenn die Reaktion innerhalb eines Wärmetauschers, vorzugsweise innerhalb eines Rohrbündelwärmetauschers abläuft. Je nach zu produzierender Produktmenge kann die Kapazität eines entsprechenden Wärmetauschers unterschiedlich gewählt werden. Für großtechnische Verfahren haben sich insbesondere Wärmetauscher mit einem Volumen von etwa 10 bis etwa 40 m³ als besonders geeignet erwiesen. Bei den bevorzugt eingesetzten Rohrbündelwärmetauschern handelt es sich um Wärmetauscher die in einen flüssigkeitsdurchströmten Mantel ein flüssigkeitsdurchströmtes Rohrbündel aufweisen. Je nach Rohrdurchmesser, Packungsdichte, etc. kann der Wärmeübergang zwischen den beiden Flüssigkeiten entsprechend eingestellt werden. Es ist im Rahmen des beschriebenen Verfahrens grundsätzlich möglich, die Reaktion dahingehend zu führen, dass das Reaktionsgemisch durch den Wärmetauscher im Rohrbündel selbst gefahren wird und die Reaktion innerhalb des Rohrbündels stattfindet, wobei die Wärme aus dem Rohrbündel in die Mantelflüssigkeit abgeführt wird.

Es hat sich jedoch ebenfalls als praktikabel und in vielen Fällen als sinnvoll erwiesen, das Reaktionsgemisch durch den Mantel des Wärmetauschers zu führen, während die zur Kühlung eingesetzte Flüssigkeit innerhalb des Rohrbündels zirkuliert. Dabei hat es sich in vielen Fällen als vorteilhaft erwiesen, wenn die Reaktionsmischung im Mantel zur Erzielung einer höheren Verweildauer und einer besseren Durchmischung über Strömungswiderstände, vorzugsweise Umlenkbleche, verteilt wird.

Das Verhältnis von Mantelvolumen zum Volumen des Rohrbündels kann dabei, je nach Auslegung des Reaktors, etwa 10 zu 1 bis etwa 1 zu 10 betragen, vorzugsweise ist das Volumen des Mantels größer als das Volumen des Rohrbündels (bezogen auf den Inhalt der Rohre).

Die Wärmeabfuhr aus dem Reaktor wird mit einem entsprechenden Kühlmittel, beispielsweise mit Wasser, so eingestellt, dass die Reaktionstemperatur innerhalb eines Korridors etwa 25 bis etwa 45°C, insbesondere bei etwa 30 bis etwa 38, insbesondere bei etwa 33 bis etwa 35°C liegt.

Aus dem Schlaufenreaktor wird kontinuierlich ein Produkt abgeführt. Das Produkt weist eine Temperatur im Rahmen der oben genannten Reaktionstemperaturen, beispielsweise eine Temperatur von etwa 35°C auf. Das Produkt wird über einen oder mehrere Wärmetauscher, insbesondere über einen oder mehrere Plattenwärmetauscher gekühlt. Dabei kommt beispielsweise eine Solekühlung zum Einsatz. Die Temperatur des Produktes nach Kühlung soll etwa 0 bis 10, insbesondere 1 bis etwa 5°C betragen. Das Produkt wird vorzugsweise in einen Lagerbehälter überführt, der eine Pufferfunktion aufweist. Zusätzlich kann das Produkt im Lagerbehälter beispielsweise durch ständiges Abführen eines Teilstroms aus dem Lagerbehälter zu einem geeigneten Wärmetauscher, beispielsweise zu einem Plattenwärmetauscher, weiter gekühlt werden, bzw. auf einer geeigneten Lagertemperatur gehalten werden. Es ist durchaus möglich, dass in dem Lagerbehälter eine Nachreaktion stattfinden kann.

Die Rückführung des Produktes in den Lagerbehälter, kann grundsätzlich auf beliebige Weise erfolgen. Es hat sich jedoch in einigen Fällen als vorteilhaft herausgestellt, dass das Produkt derart über ein System aus einer oder mehreren Düsen in den Lagerbehälter rückgeführt wird, dass innerhalb des Lagerbehälters eine entsprechende Durchmischung des gelagerten Produktes stattfindet.

Aus dem Lagerbehälter wird weiterhin kontinuierlich Produkt in einen Stabilisierungsbehälter abgeführt. Dort wird das Produkt mit einer geeigneten Säure, beispielsweise mit H₂SO₄, versetzt. Dabei wird der Katalysator deaktiviert und das Reaktionsgemisch auf einen pH-Wert von etwa 1 bis etwa 3, insbesondere etwa 2 eingestellt. Als Säure eignet sich insbesondere Schwefelsäure, beispielsweise Schwefelsäure mit einem Gehalt von etwa 90 bis etwa 105 %, insbesondere von etwa 93 bis etwa 98 % H₂SO₄.

Das stabilisierte Produkt wird dem Stabilisierungsbehälter entnommen und in die Aufreinigungsstufe überführt. Dabei kann ein Teil des entnommenen, stabilisierten Produktes beispielsweise derart in den Stabilisierungsbehälter zurückgeführt werden, dass eine ausreichende Durchmischung des Behälters über ein System aus einer oder mehreren Düsen gewährleistet ist.

### ACH-Aufarbeitung

Im Rahmen eines weiteren Verfahrenselementes, das im Zusammenhang mit der vorliegenden Erfindung eingesetzt werden kann, wird Acetoncyanhydrin, das in einer vorgelagerten Stufe beispielsweise aus der Umsetzung von Aceton mit Blausäure erhalten wurden, einer destillativen Aufarbeitung unterzogen. Dabei wird das stabilisierte rohe Acetoncyanhydrin über eine entsprechende Kolonne von niedrigsiedenden Bestandteilen befreit. Ein geeignetes Destillationsverfahren kann beispielsweise über nur eine Kolonne geführt werden. Es ist jedoch ebenfalls möglich, im Rahmen einer entsprechenden Aufreinigung von rohem Acetoncyanhydrin eine Kombination von zwei oder mehr Destillationskolonnen auch kombiniert mit einem Fallfilmverdampfer einzusetzen. Weiterhin können zwei oder mehrere Fallfilmverdampfer oder auch zwei oder mehrere Destillationskolonnen miteinander kombiniert werden.

Das rohe Acetoncyanhydrin kommt in der Regel mit einer Temperatur von etwa 0 bis etwa 15 °C, beispielsweise einer Temperatur von etwa 5 bis etwa 10°C aus der Lagerung zur Destillation. Grundsätzlich kann das rohe Acetoncyanhydrin direkt in die Kolonne eingeführt werden. Es hat sich jedoch in einigen Fällen bewährt, wenn zunächst das rohe, kühle Acetoncyanhydrin über einen Wärmetauscher einen Teil der Wärme des bereits destillativ gereinigten Produktes übernimmt. Daher wird im Rahmen einer weiteren Ausführungsform des hier beschriebenen Verfahrens das rohe Acetoncyanhydrin über einen Wärmetauscher auf eine Temperatur von etwa 60 bis 80°C erhitzt.

Die destillative Reinigung des Acetoncyanhydrin erfolgt über eine Destillationskolonne, vorzugsweise mit mehr als 10 Böden oder über eine Kaskade von zwei oder mehr entsprechend geeigneter Destillationskolonnen. Die Beheizung des Kolonnensumpfes erfolgt vorzugsweise mit Dampf. Es hat sich als vorteilhaft herausgestellt, wenn die Sumpftemperatur eine Temperatur von 140°C nicht übersteigt, gute Ausbeuten und eine gute Reinigung haben sich erzielen lassen, wenn die Sumpftemperatur nicht größer als etwa 130°C oder nicht höher als etwa 110°C ist. Die Temperaturangaben beziehen sich dabei auf die Wandtemperatur des Kolonnensumpfes.

Das rohe Acetoncyanhydrin wird im oberen Drittel der Kolonne dem Kolonnenkörper zugeführt. Die Destillation wird vorzugsweise bei verringertem Druck, beispielsweise bei einem Druck von etwa 50 bis etwa 900 mbar, insbesondere etwa 50 bis etwa 250 mbar und mit guten Ergebnissen zwischen 50 bis etwa 150 mbar durchgeführt.

Am Kopf der Kolonne werden gasförmige Verunreinigungen, insbesondere Aceton und Blausäure, entnommen, die abgetrennten gasförmigen Stoffe werden über einen Wärmetauscher oder eine Kaskade von zwei oder mehr Wärmetauschern gekühlt. Hierbei wird vorzugsweise eine Solekühlung mit einer Temperatur von etwa 0 bis etwa 10°C eingesetzt. Dabei wird den gasförmigen Inhaltsstoffen der Brüden die Gelegenheit gegeben, zu kondensieren. Die erste Kondensationsstufe kann beispielsweise bei Normaldruck stattfinden. Es ist jedoch ebenso möglich und hat sich in einigen Fällen als vorteilhaft erwiesen, wenn diese erste Kondensationsstufe unter verringertem Druck, vorzugsweise bei dem Druck, der im Rahmen der Destillation vorherrscht, erfolgt. Das Kondensat wird in einen gekühlten Auffangbehälter weitergeleitet und dort bei einer Temperatur von etwa 0 bis etwa 15°C, insbesondere bei etwa 5 bis etwa 10°C gesammelt.

Die im Rahmen des ersten Kondensationsschrittes nicht kondensierenden gasförmigen Verbindungen werden über eine Vakuumpumpe aus dem Unterdruckraum entfernt. Hierbei ist grundsätzlich eine beliebige Vakuumpumpe einsetzbar. Es hat sich jedoch in vielen Fällen als vorteilhaft erwiesen, wenn eine Vakuumpumpe eingesetzt wird, die aufgrund ihrer Bauweise nicht zum Eintrag flüssiger Verunreinigungen in den Gasstrom führt. Vorzugsweise werden hier daher beispielsweise trocken laufende Vakuumpumpen eingesetzt.

Der auf der Druckseite der Pumpe entweichende Gasstrom wird über einen weiteren Wärmetauscher geführt, der vorzugsweise mit Sole bei einer Temperatur von etwa 0 bis etwa 15°C gekühlt wird. Hierbei kondensierende Inhaltsstoffe werden ebenfalls in dem Sammelbehälter gesammelt, der bereits die unter Vakuumbedingungen gewonnenen Kondensate auffängt. Die auf der Druckseite der Vakuumpumpe durchgeführte Kondensation kann beispielsweise durch einen Wärmetauscher, jedoch auch mit einer Kaskade von zwei oder mehr seriell parallel angeordneten Wärmetauschern erfolgen. Nach diesem Kondensationsschritt verbleibende gasförmigen Stoffen werden abgeführt und einer beliebigen weiteren Verwertung, beispielsweise einer thermischen Verwertung, zugeführt.

Die gesammelten Kondensate können ebenfalls beliebig weiterverwertet werden. Es hat sich jedoch als unter ökonomischen Gesichtspunkten äu-βerst Vorteilhaft erwiesen, die Kondensate in die Reaktion zur Herstellung von Acetoncyanhydrin zurückzuführen. Dies erfolgt vorzugsweise an einer oder mehreren Stellen, die Zugang zum Schlaufenreaktor ermöglichen. Die Kondensate können grundsätzlich eine beliebige Zusammensetzung aufweisen, sofern sie die Herstellung des Acetoncyanhydrins nicht stören. In vielen Fällen wird die überwiegende Menge des Kondensates jedoch aus Aceton und Blausäure bestehen, beispielsweise in einem molaren Verhältnis von etwa 2:1 bis etwa 1:2, häufig in einem Verhältnis von etwa 1:1.

Das aus dem Sumpf der Destillationskolonne gewonnene Acetoncyanhydrin wird zunächst über einen ersten Wärmetauscher durch das zugeführte, kalte rohe Acetoncyanhydrin auf eine Temperatur von etwa 40 bis etwa 80°C abgekühlt. Anschließend wird das Acetoncyanhydrin über einen mindestens einen weiteren Wärmetauscher auf eine Temperatur von etwa 30 bis etwa 35 °C gekühlt und ggf. zwischengelagert.

### Amidierung

Im Rahmen eines weiteren Verfahrenselements, wie er häufig bei der Herstellung von Methacrylsäure oder von Estern der Methacrylsäure vorgesehen ist, wird Acetoncyanhydrin einer Hydrolyse unterzogen. Dabei bildet sich bei verschiedenen Temperaturstufen nach einer Reihe von Reaktionen als Produkt Methacrylamid.

Die Umsetzung wird in einer dem Fachmann bekannten Weise durch eine Reaktion zwischen konzentrierter Schwefelsäure und Acetoncyanhydrin bewirkt. Die Umsetzung ist exotherm, so dass in vorteilhafter Weise Reaktionswärme aus dem System abgeführt wird.

Die Umsetzung kann auch hier wieder im Batchverfahren oder in kontinuierlichen Verfahren durchgeführt werden. Letzteres hat sich in vielen Fällen als vorteilhaft erwiesen. Sofern die Umsetzung im Rahmen eines kontinuierlichen Verfahrens durchgeführt wird, hat sich der Einsatz von Schlaufenreaktoren bewährt. Die Umsetzung kann beispielsweise in nur einem Schlaufenreaktor erfolgen. Es kann jedoch vorteilhaft sein, wenn die Umsetzung in einer Kaskade von zwei oder mehreren Schlaufenreaktoren durchgeführt wird.

Ein geeigneter Schlaufenreaktor weist im Rahmen des beschriebenen Verfahrens eine oder mehrere Zufuhrstellen für Acetoncyanhydrin, eine oder mehrere Zufuhrstellen für konzentrierte Schwefelsäure, einen oder mehrere Gasabscheider, einen oder mehrere Wärmetauscher und eine oder mehrere Mischer und oftmals eine Pumpe als ein Fördermittel auf.

Die Hydrolyse von Acetoncyanhydrin mit Schwefelsäure zu Methacrylamid ist, wie bereits beschrieben, exotherm. Die im Rahmen der Reaktion anfallende Reaktionswärme muss dem System jedoch zumindest weitgehend entzogen werden, da mit zunehmender Temperatur bei der Umsetzung die Ausbeute sinkt. Zwar ist es grundsätzlich möglich mit entsprechenden Wärmetauschern eine schnelle und umfassende Abfuhr der Reaktionswärme zu erzielen. Es kann jedoch auch nachteilig sein, das Gemisch zu sehr zu kühlen, da für einen entsprechenden Austausch an den Wärmetauschern ein ausreichender Wärmeübergang erforderlich ist. Da mit sinkender Temperatur die Viskosität des Gemischs stark ansteigt, wird so einerseits die Zirkulation im bzw. der Durchfluss durch den Schlaufenreaktor erschwert, andererseits lässt sich eine ausreichende Abfuhr der Reaktionsenergie aus dem System nicht mehr gewährleisten.

Darüber hinaus können zu geringe Temperaturen im Reaktionsgemisch zu einer Kristallisation von Inhaltsstoffen des Reaktionsgemischs an den Wärmetauschern führen. Dadurch wird der Wärmeübergang weiter verschlechtert, wodurch sich eindeutiger Ausbeuterückgang verzeichnen lässt. Darüber hinaus kann der Schlaufenreaktor nicht mit den optimalen Mengen an Reaktanden beschickt werden, so dass insgesamt die Effizienz des Verfahrens leidet.

Im Rahmen einer Ausgestaltung des Verfahrens wird aus einem Strom von Acetoncyanhydrin ein Teil, vorzugsweise etwa zwei Drittel bis etwa drei Viertel, des Volumenstroms in einen ersten Schlaufenreaktor eingeführt. Vorzugsweise weist ein erster Schlaufenreaktor einen oder mehrere Wärmetauscher, eine oder mehrere Pumpen, einen oder mehrere Mischelemente und einen oder mehrere Gasabscheider auf. Die den ersten Schlaufenreaktor durchlaufenden Umwältsströme liegen vorzugsweise im Bereich von etwa 50 bis 650 m³/h, bevorzugt in einem Bereich von 100 bis 500 m³/h und darüber hinaus bevorzugt in einem Bereich von etwa 150 bis 450 m³/h. In einem auf den ersten Schlaufenreaktor folgenden mindestens einem weiteren Schlaufenreaktor liegen die Umwältzströme vorzugsweise in einem Bereich von etwa 40 bis 650 m³/h, bevorzugt in einem Bereich von 50 bis 500 m³/h und darüber hinaus bevorzugt in einem Bereich von etwa 60 bis 350 m³/h. Weiterhin sind als Temperaturdifferenz über den Wärmetauscher etwa 1 bis 20° C bevorzugt, wobei etwa 2 bis 7°C besonders bevorzugt sind.

Die Zufuhr des Acetoncyanhydrin kann grundsätzlich an beliebiger Stelle in den Schlaufenreaktor erfolgen. Es hat sich jedoch als vorteilhaft erwiesen, wenn die Zufuhr in ein Mischelement, beispielsweise in einen Mischer mit beweglichen Teilen oder einen Statikmischer oder an gut durchmischter Stelle erfolgt. Die Zufuhr der Schwefelsäure erfolgt vorteilhafter Weise vor der Acetoncyanhydrinzugabe. Ansonsten ist es jedoch ebenfalls möglich, die Schwefelsäure an beliebiger Stelle in den Schlaufenreaktor einzuführen.

Das Verhältnis der Reaktanden im Schlaufenreaktor wird so gesteuert, dass ein Überschuss Schwefelsäure vorliegt. Der Überschuss an Schwefelsäure beträgt, bezüglich des molaren Verhältnisses der Inhaltsstoffe, im ersten Schlaufenreaktor etwa 1,8:1 bis etwa 3:1 und im letzten Schlaufenreaktor etwa 1,3:1 bis etwa 2:1.

In einigen Fällen hat es sich als vorteilhaft erwiesen, mit einem derartigen Überschuss an Schwefelsäure die Reaktion im Schlaufenreaktor zu betreiben. Die Schwefelsäure kann hier beispielsweise als Lösemittel dienen und die Viskosität des Reaktionsgemischs niedrig halten, wodurch eine höhere Abfuhr von Reaktionswärme und eine niedrigere Temperatur des Reaktionsgemischs gewährleistet werden kann. Dies kann deutliche Ausbeutevorteile mit sich bringen. Die Temperatur im Reaktionsgemisch beträgt etwa 90 bis etwa 120°C.

Die Wärmeabfuhr wird durch einen oder mehrere Wärmetauscher im Schlaufenreaktor gewährleistet. Es hat sich dabei als vorteilhaft erwiesen, wenn die Wärmetauscher über eine geeignete Sensorik zum Einstellen der Kühlleistung verfügen, um eine zu starke Kühlung des Reaktionsgemischs aus den oben genannten Gründen zu verhindern. So kann es beispielsweise vorteilhaft sein, den Wärmeübergang im Wärmetauscher oder in den Wärmetauschern punktförmig oder kontinuierlich zu messen und daran die Kühlleistung der Wärmetauscher anzupassen. Dies kann beispielsweise über das Kühlmittel selbst geschehen. Es ist auch ebenso möglich durch entsprechende Variation der Zugabe der Reaktionspartner und durch die Erzeugung von mehr Reaktionswärme eine entsprechenden Erhitzung des Reaktionsgemischs zu erreichen. Auch eine Kombination von beiden Möglichkeiten ist denkbar. Der Schlaufenreaktor sollte darüber hinaus über mindestens einen Gasabscheider verfügen. Über den Gasabscheider wird einerseits dem Schlaufenreaktor kontinuierlich gebildetes Produkt entnommen. Andererseits lassen sich im Rahmen der Reaktion gebildete Gase so dem Reaktionsraum entziehen. Als Gas bildet sich hauptsächlich Kohlenmonoxid. Das aus dem Schlaufenreaktor entnommene Produkt wird vorzugsweise in einen zweiten Schlaufenreaktor überführt. In diesen zweiten Schlaufenreaktor wird das Schwefelsäure und Metharcylsäureamid beinhaltende Reaktionsgemisch wie es durch die Reaktion im ersten Schlaufenreaktor erhalten wurde, mit dem verbleibenden Teilstrom an Acetoncyanhydrin umgesetzt. Hierbei reagiert der Überschuss an Schwefelsäure aus dem ersten Schlaufenreaktor, oder zumindest ein Teil der überschüssigen Schwefelsäure, mit dem Acetoncyanhydrin unter weiterer Bildung von Methacrylsäureamid. Die Durchführung der Reaktion in zwei oder mehr Schlaufenreaktoren weist den Vorteil auf, dass aufgrund des Schwefelsäureüberschusses im ersten Schlaufenreaktor die Pumpbarkeit des Reaktionsgemischs und damit der Wärmeübergang und letztendlich die Ausbeute verbessert werden. Im zweiten Schlaufenreaktor ist wiederum mindestens ein Mischelement, mindestens ein Wärmetauscher und mindestens ein Gasabscheider angeordnet. Die Reaktionstemperatur im zweiten Schlaufenreaktor beträgt ebenfalls etwa 90 bis etwa 120°C.

Das Problem der Pumpbarkeit des Reaktionsgemischs, des Wärmeübergangs und einer möglichst geringen Reaktionstemperatur stellt sich im jedem weiteren Schlaufenreaktor genauso wie im ersten. Deshalb verfügt vorteilhafter Weise auch der zweite Schlaufenreaktor über einen Wärmetauscher, dessen Kühlleistung durch entsprechende Sensorik geregelt werden kann.

Die Zufuhr des Acetoncyanhydrins erfolgt wiederum in einem geeigneten Mischelement, vorzugsweise in einen Statikmischer oder an gut durchmischter Stelle.

Aus dem Abscheider, insbesondere Gasabscheider, des zweiten Schlaufenreaktors wird das Produkt entnommen und zur Vervollständigung der Umsetzung und zur Bildung des Methacrylsäureamids auf eine Temperatur von etwa 130 bis etwa 180°C erhitzt.

Die Erhitzung wird vorzugsweise derart durchgeführt, dass die Maximaltemperatur nur für einen möglichst kurzen Zeitraum, beispielsweise für eine Zeit von etwa einer Minute bis etwa 30 Minuten, insbesondere für eine Zeit von etwa zwei bis etwa acht oder etwa drei bis etwa fünf Minuten erreicht wird. Dies kann grundsätzlich in beliebigen Apparaturen zur Erzielung einer derartigen Temperatur für einen derart kurzen Zeitraum erfolgen. Beispielsweise kann die Energiezufuhr auf konventionellem Wege durch elektrische Energie oder durch Dampf erfolgen. Es ist jedoch ebenso möglich, die Energie durch elektromagnetische Strahlung, beispielsweise durch Mikrowellen zuzuführen.

Es hat sich in verschiedenen Fällen als vorteilhaft erwiesen, wenn der Erhitzungsschritt in einem Wärmetauscher mit zwei oder mehrstufiger Anordnung von Rohrwendeln, die vorzugsweise in einer mindestens doppelten, gegenläufigen Anordnung vorliegen können, erfolgt. Dabei wird das Reaktionsgemisch schnell auf eine Temperatur von etwa 130 bis 180°C erhitzt.

Der Wärmetauscher kann beispielsweise mit einem oder mehreren Gasabscheidern kombiniert werden. So ist es beispielsweise möglich, das Reaktionsgemisch nach Verlassen der ersten Rohrwendel im Wärmetauscher durch einen Gasabscheider zu führen. Dabei können beispielsweise während der Reaktion entstehende, gasförmige Komponenten aus dem Reaktionsgemisch abgetrennt werden. Es ist ebenso möglich, das Reaktionsgemisch nach Verlassen der zweiten Wendel mit einem Gasabscheider zu behandeln. Es kann sich darüber hinaus als vorteilhaft erweisen, an beiden Stellen, sowohl nach Verlassen der ersten als auch nach Verlassen der zweiten Rohrwendel das Reaktionsgemisch mit einem Gasabscheider zu behandeln.

Die so erhältliche Amidlösung weist in der Regel eine Temperatur von mehr als 100°C, üblicherweise eine Temperatur von etwa 130 bis 180°C auf.

Die im Rahmen der Amidierung anfallenden gasförmigen Verbindungen können grundsätzlich in beliebiger Weise entsorgt oder einer Weiterverarbeitung zugeführt werden. Es kann in einigen Fällen jedoch vorteilhaft sein, wenn die entsprechenden Gase in einer Transportleitung dergestalt zusammengeführt werden, dass sie entweder kontinuierlich oder bei Bedarf ggf. mit Druck, beispielsweise mit Dampfdruck, beaufschlagt und so weitertransportiert werden können.

### Veresterung

Ein weiterer ein Verfahrenselement darstellender Schritt, der im Rahmen der vorliegenden Erfindung im Zusammenhang mit dem erfindungsgemäßen Verfahren eingesetzt werden kann, ist eine Hydrolyse von Methacrylsäureamid zu Methacrylsäure und deren gleichzeitiger Veresterung zu Methacrylsäureester. Diese Reaktion kann in einem oder mehreren beheizten, beispielsweise durch Dampf beheizten Kesseln, durchgeführt werden. Es hat sich in vielen Fällen als vorteilhaft erwiesen, wenn die Veresterung in mindestens zwei aufeinander folgenden Kesseln, beispielsweise jedoch auch in drei oder vier oder mehr aufeinander folgenden Kesseln durchgeführt wird. Dabei wird eine Lösung von Methacrylsäureamid in den Kessel bzw. in den ersten Kessel einer zwei oder mehr Kessel umfassenden Kaskade von Kesseln eingeführt.

Es ist häufig bevorzugt, eine entsprechende Veresterungsreaktion mit einer Kaskade von zwei oder mehr Kesseln durchzuführen. Im Folgenden soll daher ausschließlich auf diese Variante Bezug genommen werden.

Im Rahmen des hier beschriebenen Verfahrens kann beispielsweise eine Amidlösung, wie sie aus der hier beschriebenen Amidierungsreaktion erhältlich ist, in einen ersten Kessel eingespeist werden. Der Kessel wird beispielsweise mit Dampf beheizt. Die zugeführte Amidlösung weist in der Regel eine erhöhte Temperatur auf, beispielsweise eine Temperatur von etwa 100 bis etwa 180°C, im Wesentlichen entsprechend der Ablauftemperatur der Amidlösung aus der oben vorgestellten Amidierungsreaktion. Den Kesseln wird weiterhin ein Alkanol zugeführt, das zur Veresterung eingesetzt werden kann.

Grundsätzlich eignen sich hier beliebige Alkanole mit 1 bis etwa 4 Kohlenstoffatomen, die linear oder verzweigt, gesättigt oder ungesättigt sein können, wobei Methanol besonders bevorzugt ist. Ebenso können diese Alkanole zusammen mit Methacrylsäureestern eingesetzt werden, was insbesondere bei Umesterungen der Fall ist.

Der Kessel wird weiterhin mit Wasser beschickt, so dass insgesamt eine Wasserkonzentration im Kessel von etwa 13 bis etwa 26 Gew.-%, insbesondere etwa 18 bis etwa 20 Gew.-% herrscht.

Die Menge an Amidlösung und an Alkanol wird derart geregelt, dass ein Gesamtmolverhältnis von Amid zu Alkanol von etwa 1:1,4 bis etwa 1:1,6 herrscht. Das Alkanol kann auf die Kesselkaskade derart verteilt werden, dass im ersten Reaktor das Molverhältnis etwa 1:1,1 bis etwa 1:1,4 beträgt und in den folgenden Reaktionsstufen bezogen auf den Gesamtamidstrom Molverhältnisse von etwa 1:0,05 bis etwa 1:0,3 eingestellt werden. Das in die Veresterung zugeführte Alkanol kann sich aus "Frischalkanol" sowie Alkanol aus Recyclingströmen der Aufarbeitungsstufen und bei Bedarf auch aus Recyclingströmen der Downstreamprozesse des Produktionsverbundes zusammen setzen.

Die Beschickung des ersten Kessels mit Wasser kann grundsätzlich dahingehend erfolgen, dass Wasser aus einer beliebigen Quelle dem Kessel zugeführt wird, sofern dieses Wasser keine Inhaltsstoffe aufweist, die die Veresterungsreaktion oder die nachfolgenden Prozeßstufen nachteilig beeinflussen könnten. Beispielsweise kann dem Kessel VE-Wasser oder Brunnenwasser zugeführt werden. Es ist jedoch ebenfalls möglich, dem Kessel ein Gemisch aus Wasser und organischen Verbindungen zuzuführen, wie sie beispielsweise bei der Reinigung von Methacrylsäure oder Methacrylsäureestern anfallen. Im Rahmen einer bevorzugten Ausführungsform des hier vorgestellten Verfahrens werden die Kessel zumindest anteilig mit einem Gemisch aus Wasser und solchen organischen Verbindungen beschickt.

Wenn eine Kaskade von zwei oder mehr Kesseln im Rahmen der Veresterungsreaktion eingesetzt wird, so können die entstandenen gasförmigen Stoffe, insbesondere der Methacrylsäureester, grundsätzlich aus jedem Kessel einzeln abgezogen und einer Reinigung zugeführt werden. Es hat sich jedoch in manchen Fällen als vorteilhaft erwiesen, wenn bei einer Kaskade von zwei oder mehr Kesseln die gasförmigen Produkte aus dem ersten Kessel zunächst in den zweiten Reaktionskessel eingespeist werden, ohne dass die gasförmigen Verbindungen aus dem ersten Kessel direkt einer Reinigung zugeführt werden. Diese Vorgehensweise bietet den Vorteil, dass der im ersten Kessel häufig starken Schaumentwicklung nicht durch aufwendige, apparative Entschäumung begegnet werden muss. Im Falle einer Kaskadierung der gasförmigen Stoffe aus dem ersten Kessel in den zweiten Kessel tritt der im ersten Kessel gebildete und ggf. mitgerissene Schaum einfach in den Reaktionsraum des zweiten Kessels mit ein. Da dort in der Regel die Schaumbildung deutlich geringer ist, muss so nicht apparativ entschäumt werden.

Der nach einem ersten Kessel angeordnete zweite Kessel nimmt nun einerseits den Überlauf des ersten Kessels auf, andererseits wird er mit den gasförmigen im ersten Kessel gebildeten oder im ersten Kessel vorhandenen Stoffen gespeist. Der zweite Kessel und die evt. folgenden werden ebenfalls mit Methanol beschickt. Hierbei ist es bevorzugt, dass die Methanolmenge von Kessel zu Kessel um mindestens 10 %, jeweils auf den vorhergehenden Kessel bezogen, abnimmt. Die Wasserkonzentration im zweiten Kessel sowie in den weiteren Kesseln kann sich von der des ersten Kessels unterscheiden, oftmals sind die Konzentrationsunterschiede jedoch gering.

Die im zweiten Kessel entstehenden Brüden werden aus dem Kessel abgeführt und in den Sumpf einer Destillationskolonne eingeleitet.

Wenn die Veresterung mit einer Kaskade von drei oder mehr Kesseln durchgeführt wird, so wird jeweils der Überlauf des zweiten Kessels in einen dritten Kessel überführt sowie der Überlauf des dritten Kessels ggf. in einen vierten Kessel überführt. Die weiteren Kessel werden ebenfalls dampfbeheizt. Vorzugsweise wird die Temperatur in den Kesseln 3 und ggf. 4 auf etwa 120 °C bis etwa 140 °C eingestellt.

Die aus den Kesseln entweichenden Brüden werden in eine Destillationskolonne eingeleitet, wobei diese vorzugsweise im Unterbereich der Destillationskolonne erfolgt. Die Brüden umfassen ein azeotropes Gemisch aus Träger-Dampf, Methacrylsäureester und Alkanol und weisen je nach eingesetztem Alkanol eine Temperatur von etwa 60 bis etwa 120°C, beispielsweise etwa 70 bis etwa 90°C bei Einsatz von Methanol auf. In der Destillationskolonne wird der Methacrylsäureester gasförmig von den bei höheren Temperaturen siedenden Brüdenbestandteilen abgetrennt. Die hochsiedenden Anteile (hauptsächlich Methacrylsäure, Hydroxyisobuttersäureester und Wasser) werden in den ersten Reaktionskessel zurückgeführt. Der gebildete Methacrylsäureester wird am Kolonnenkopf abgezogen und über einen Wärmetauscher oder eine Kaskade von zwei oder mehr Wärmetauschern abgekühlt. Es hat sich in einigen Fällen bewährt, wenn die Kühlung des Methacrylsäureesters über mindestens zwei Wärmetauscher erfolgt, wobei ein erster Wärmetauscher mit Wasser die Kondensation und eine Kühlung auf eine Temperatur von etwa 60 bis etwa 30°C durchführt, während ein zweiter, solegekühlter Wärmetauscher eine Abkühlung auf etwa 5 bis etwa 15°C vornimmt. Von dem wassergekühlten Kondensat kann ein Teilstrom als Rücklauf auf die Kolonnen zur Konzentrationssteuerung der Kolonne gegeben. Es ist jedoch ebenso möglich, den gebildeten Methacrylsäureester über eine Kaskade von mehr als zwei Wärmetauschern zu kühlen. Hierbei ist es beispielsweise möglich, zunächst eine Kühlung über zwei hintereinander geschaltete, wassergekühlte Wärmetauscher vorzunehmen und anschließend über einen entsprechenden solegekühlten Wärmetauscher eine weitere Abkühlung zu erzielen.

So kann beispielsweise im Rahmen des hier vorgestellten Verfahrens der gebildete Methacrylsäureester in gasförmigem Zustand über einen ersten Wärmetauscher mit Wasserkühlung gekühlt werden. Sowohl kondensierte als auch nicht kondensierte Stoffe werden anschließend in einen zweiten Wärmetauscher weitergeleitet, wo eine weitere Kondensation über Wasserkühlung stattfindet. An dieser Stelle können nun beispielsweise gasförmige Stoffe in einen separaten, mit Sole gekühlten Wärmetauscher überführt werden. Das Kondensat in diesem solegekühlten Wärmetauscher wird anschließend in den Destillatstrom gegeben, während die verbleibenden gasförmigen Stoffe weiter verwertet werden können oder einer Entsorgung zugeführt werden. Das Methacrylsäureester-Kondensat aus dem zweiten wassergekühlten Wärmetauscher wird nun in einem mit Wasser oder mit Sole gekühlten Wärmetauscher auf eine Temperatur von weniger als 15°C, vorzugsweise etwa 8 bis etwa 12°C gekühlt. Dieser Kühlschritt kann dazu führen, dass der gebildete Methacrylsäureester einen deutlich niedrigeren Gehalt an Ameisensäure aufweist, als dies ohne den entsprechenden Kühlungsschritt der Fall wäre. Das abgekühlte Kondensat wird anschließend in einen Phasentrenner überführt. Hier wird die organische Phase (Methacrylsäureester) von der wässrigen Phase getrennt. Die wässrige Phase, die neben Wasser noch einen Gehalt an organischen Verbindungen, insbesondere Alkanol, aus dem Destillationsschritt aufweisen kann, kann grundsätzlich beliebig weiterverwendet werden. Wie jedoch bereits oben beschrieben, kann es bevorzugt sein, dieses Gemisch aus Wasser und organischen Verbindungen wieder in den Veresterungsprozess zurückzuführen, indem eine Einspeisung in den ersten Reaktionskessel erfolgt.

Die abgetrennte organische Phase wird in einen Wäscher eingespeist. Dort wird der Methacrylsäureester mit demineralisiertem Wasser gewaschen. Die abgeschiedene wässrige Phase, die ein Gemisch aus Wasser und organischen Verbindungen, insbesondere Alkanol, enthält, kann wiederum grundsätzlich beliebig weiterverwendet werden. Es ist jedoch unter ökonomischen Gesichtspunkten vorteilhaft, diese wässrige Phase wieder in den Veresterungsschritt rückzuführen, indem sie beispielsweise in den ersten Kessel eingespeist wird.

Da Methacrylsäureester eine starke Neigung zur Polymerisation aufweisen, ist es in vielen Fällen vorteilhaft, wenn im Rahmen der Veresterung von Methacrylsäure dafür Sorge getragen wird, dass eine derartige Polymerisation verhindert wird.

In Anlagen zur Herstellung von Methacrylsäure oder Methacrylsäureestern findet Polymerisation oft dann statt, wenn Methacrylsäure oder Methacrylsäureester einerseits eine geringe Strömungsgeschwindigkeit aufweisen, so dass sich lokal Ruhezonen ausbilden können, in denen ein über längere Zeit andauernder Kontakt zwischen Methacrylsäure oder Methacrylsäureester und einem Polymerisationsinitiator einstellen kann, der in Folge dann zur Polymerisation führen kann.

Um ein entsprechendes Polymerisationsverhalten zu vermeiden, kann es vorteilhaft sein, eine Optimierung des Stoffflusses dahingehend durchzuführen, dass einerseits die Strömungsgeschwindigkeit des Methacrylsäureesters oder der Methacrylsäure an möglichst allen Stellen im System so hoch ist, dass die Zahl der Ruhezonen minimiert wird. Darüber hinaus kann es vorteilhaft sein, den Strom an Methacrylsäure bzw. Methacrylsäureester derart mit geeigneten Stabilisatoren zu versetzen, dass eine Polymerisation weitgehend unterdrückt wird.

Zu diesem Zweck können im Rahmen des hier dargestellten Verfahrens grundsätzlich die Stoffströme derart mit Stabilisatoren versetzt werden, dass möglichst wenig Polymerisation im System selbst stattfindet. Hierzu wird insbesondere der Teil der Anlage mit entsprechenden Stabilisatoren versorgt, in dem die Methacrylsäure bzw. der Methacrylsäureester während oder nach der Destillation in hoher Konzentration vorliegt.

So hat es sich beispielsweise als sinnvoll erwiesen, am Kopf der Destillationskolonne dem dort abgezogenen Strom an Methacrylsäureester einen Stabilisator zuzuführen. Weiterhin hat es sich als vorteilhaft erwiesen, diejenigen anlagenteile mit einer Lösung von Stabilisator in Methacrylsäureester zu spülen, in denen Methacrylsäure oder Methacrylsäureester mit einer Temperatur von mehr als etwa 20°C, vorzugsweise mit einer Temperatur im Bereich von etwa 20 bis etwa 120°C zirkuliert. So wird beispielsweise ein Teil des in den Wärmetauschern anfallenden Kondensates zusammen mit einem geeigneten Stabilisator derart in den Kopf der Destillationskolonne zurückgeführt, dass dort der Kolonnenkopf an seiner Innenseite ständig mit stabilisiertem Methacrylsäureester oder stabilisierter Methacrylsäure besprüht wird. Dies geschieht vorzugsweise derart, dass sich im Kolonnenkopf keine Ruhezonen ausbilden können, an denen eine Polymerisation von Methacrylsäure oder Methacrylsäureester zu befürchten ist. Die Wärmetauscher selbst können entsprechend ebenfalls mit einer stabilisierten Lösung von Methacrylsäure oder Methacrylsäureester derart beaufschlagt werden, dass auch hier keine Ruhezonen zur Ausbildung gelangen können.

Es hat sich weiterhin als Vorteil im Rahmen des hier vorgestellten Verfahrens gezeigt, wenn beispielsweise die Kohlenmonoxid enthaltenden Abgase aus vorangehenden Prozessen, insbesondere aus dem Amidierungsschritt, zusammen mit Dampf durch die Veresterungsanlage geleitet werden. Auf diese Weise erfolgt eine nochmalige Vereinigung des Gasgemisches von Verbindungen, die als Feststoff oder als Flüssigkeit abgetrennt werden können. Zum anderen werden diese an einer zentralen Stelle gesammelt und können der weiteren Verwertung oder Entsorgung zugeführt werden.

Das im Rahmen der Veresterung und der anschließenden Vorreinigung erhaltene MMA bzw. der erhaltene Methacrylsäureester oder die erhaltene Methacrylsäure werden anschließend einer weiteren Behandlung zugeführt. Aus der Veresterung resultiert als verbleibender Reststoff verdünnte Schwefelsäure, die ebenfalls einer weiteren Verwertung zugeführt werden kann.

### Vorreinigung des Esters oder der Säure

Im Rahmen des hier vorgestellten Verfahrens kann der Gegenstand der vorliegenden Erfindung auch im Zusammenhang mit einem Verfahren zur Vorreinigung von Methacrylsäure oder Methacrylsäureester eingesetzt werden, wie dieses in dem nachfolgenden Verfahrenselement beschrieben wird. So wird grundsätzlich rohe Methacrylsäure oder ein roher Methacrylsäureester einer weiteren Reinigung unterzogen, um zu einem möglichst reinen Produkt zu gelangen. Eine derartige ein weiteres Verfahrenselement darstellende Reinigung kann beispielsweise einstufig sein. Es hat sich jedoch in vielen Fällen als vorteilhaft herausgestellt, wenn eine solche Reinigung mindestens zwei Stufen umfasst, wobei in einer ersten Vorreinigung, wie sie hier beschrieben wird, die niedrigsiedenden Bestandteile des Produktes entfernt werden. Hierzu wird roher Methacrylsäureester bzw. rohe Methacrylsäure zunächst in eine Destillationskolonne überführt, in welcher die niedrigsiedenden Bestandteile und Wasser abgetrennt werden können. Hierzu wird der rohe Methacrylsäureester einer Destillationskolonne zugeführt, wobei die Zugabe etwa in der oberen Hälfte der Kolonne durchgeführt wird. Der Kolonnensumpf wird mit Dampf beispielsweise derart beheizt, dass eine Wandtemperatur von etwa 50 bis etwa 120°C erreicht wird. Die Reinigung wird unter Vakuum durchgeführt. Der Druck innerhalb der Kolonne beträgt im Fall des Esters vorzugsweise etwa 100 bis etwa 600 mbar. Der Druck innerhalb der Kolonne beträgt im Fall der Säure vorzugsweise etwa 40 bis etwa 300 mbar.

Am Kolonnenkopf werden die niedrigsiedenden Bestandteile abgenommen. Insbesondere können dieses beispielsweise Ether, Aceton und Methylformiat sein. Die Brüden werden anschließend über einen oder mehrere Wärmetauscher kondensiert. Dabei hat es sich beispielsweise in einigen Fällen bewährt, zunächst eine Kondensation über zwei in Serie geschaltete, mit Wasser gekühlte Wärmetauscher durchzuführen. Es ist jedoch ebenso möglich, an dieser Stelle auch nur einen Wärmetauscher einzusetzen. Die Wärmetauscher werden vorzugsweise zur Erhöhung der Flussgeschwindigkeit und um die Ausbildung stationärer Phasen zu verhindern, in senkrechtem Zustand betrieben, wobei es bevorzugt ist, eine möglichst vollständige Benetzung zu erhalten. Dem wassergekühlten Wärmetauscher oder den wassergekühlten Wärmetauschern nachgeschaltet, kann ein solegekühlter Wärmetauscher sein, es ist jedoch auch möglich, eine Kaskade von zwei oder mehr solegekühlten Wärmetauschern nachzuschalten. In der Kaskade von Wärmetauschern werden die Brüden kondensiert, mit Stabilisator versehen und beispielsweise einem Phasentrenner zugeführt. Da die Brüden auch Wasser enthalten können, wird eine evtl. anfallende wässrige Phase entsorgt oder einer weiteren Verwertung zugeführt. Als weitere Verwertung bietet sich beispielsweise die Rückführung in eine Veresterungsreaktion, beispielsweise in eine Veresterungsreaktion wie sie oben beschrieben wurde, an. In diesem Fall wird die wässrige Phase vorzugsweise in den ersten Veresterungskessel rückgeführt.

Die abgetrennte organische Phase wird als Rückfluß in den Kolonnenkopf eingespeist. Ein Teil der organischen Phase kann wiederum zum Besprühen der Wärmetauscherköpfe und des Kolonnenkopfes eingesetzt werden. Da es sich bei der abgetrennten organischen Phase um eine Phase handelt, die mit Stabilisator versetzt ist, lässt sich so wirksam einerseits die Ausbildung von Ruhezonen verhindern. Andererseits bewirkt die Gegenwart des Stabilisators eine weitere Unterbindung der Polymerisationsneigung der abgetrennten Brüden.

Der aus den Wärmetauschern erhaltene Kondensatstrom wird darüber hinaus vorzugsweise derart mit demineralisiertem Wasser versetzt, dass im Phasentrenner eine ausreichende Abscheidwirkung erzielt werden kann.

Die nach der Kondensation in der Wärmetauscherkaskade verbleibenden, gasförmigen Verbindungen können, vorzugsweise mittel Dampfstrahler als Unterdruckerzeuger, nochmals über einen oder mehrere weitere Wärmetauscher einer Kondensation unterzogen werden. Es hat sich dabei unter ökonomischen Gesichtspunkten als vorteilhaft herausgestellt, wenn im Rahmen einer solchen Nachkondensation nicht nur die gasförmigen Stoffe aus der Vorreinigung kondensiert werden. So ist es beispielsweise möglich, einer derartigen Nachkondensation weitere gasförmige Stoffe zuzuführen, wie sie sich aus der Hauptreinigung von Methacrylsäureestern ergeben. Der Vorteil einer derartigen Verfahrensweise liegt beispielsweise darin, dass so ein Anteil an Methacrylsäureester, der im Rahmen der Hauptreinigungsstufe nicht kondensiert wurde, im Rahmen der Vorreinigung nochmals über den Phasenabscheider in die Reinigungskolonne überführt werden kann. So wird beispielsweise gewährleistet, dass eine Ausbeutemaximierung stattfinden kann, und möglichst geringe Verluste an Methacrylsäureester auftreten. Außerdem kann durch die geeignete Wahl der Auslegung und des Betriebs dieser weiteren Wärmetauscher die Zusammensetzung des diese Wärmetauscher verlassenden Abgases, insbesondere der Gehalt an Leichtsiedern, eingestellt werden.

Aufgrund der Zuführung von Wasser im Rahmen der Vorreinigung des Methacrylsäureesters kann der Wassergehalt in der Veresterung und die Konzentration an niedrigsiedenden Bestandteilen im Roh-Methylmethacrylat insgesamt kontinuierlich ansteigen. Um dies zu vermeiden, kann es vorteilhaft sein, einen Teil des dem System zugeführten Wassers, vorzugsweise kontinuierlich, aus dem System auszuschleusen. Dieses Ausschleusen kann grundsätzlich beispielsweise in einer Größenordnung erfolgen, in der in der Vorreinigung dem System Wasser zugeführt wird. Die im Phasentrenner abgeschiedene wässrige Phase weist üblicherweise einen Gehalt an organischen Inhaltsstoffen auf. Es kann daher vorteilhaft sein, dieses Wasser einer Form der Entsorgung zuzuführen, die diesen Gehalt an organischen Stoffen ausnutzt.

So kann es beispielsweise vorteilhaft sein, wenn ein derart mit organischen Stoffen belastetes Wasser im Rahmen eines Schwefelsäurespaltverfahrens dem Brennraum zugemischt wird. Aufgrund der oxidierbaren Inhaltsstoffe kann so deren Brennwert, zumindest teilweise, noch genutzt werden. Darüber hinaus wird so eine möglicherweise kostspielige Entsorgung des mit organischen Stoffen belasteten Wassers oft vermieden.

### Feinreinigung des Methacrylsäureesters

Zur Feinreinigung des Methacrylsäureesters wird der rohe, vorgereinigte Methacrylsäureester einer erneuten Destillation unterzogen. Dabei wird der rohe Methacrylsäureester mit Hilfe einer Destillationskolonne von seinen hochsiedenden Bestandteilen befreit und so ein Reinmethacrylsäureester erhalten. Hierzu wird der rohe Methacrylsäureester in einer dem Fachmann bekannten Weise, mitunter in die untere Hälfte, einer Destillationskolonne eingebracht.

Die Destillationskolonne kann grundsätzlich einer beliebigen, dem Fachmann geeignet erscheinenden Ausführung entsprechen. Es hat sich jedoch für die Reinheit des erhaltenen Produkts in vielen Fällen als vorteilhaft herausgestellt, wenn die Destillationskolonne mit einer oder mehreren Packungen betrieben wird, die den folgenden Vorgaben etwa enspricht:
Zum einen sollen sich in den Kolonnen genauso wie in den anderen mit Methacrylsäureester durchströmten Leitungen möglichst wenig so genannte "Toträume" bilden. Die Toträume führen zu einer vergleichsweise langen Verweilzeit der Methacrylsäureester, die deren Polymerisation begünstigen. Dieses führt wiederum zu kostspieligen Produktionsunterbrechungen und Reinigungen der entsprechenden mit Polymer zugesetzten Teile. Der Bildung von Toträumen kann unter anderem dadurch begegnet werden, dass sowohl durch Auslegung als auch durch eine geeignete Betriebsweise der Kolonnen, diese stets mit einer ausreichenden Menge von Flüssigkeit beladen werden, so dass eine ständige Umspülung der Kolonnen und insbesondere der Kolonneneinbauten wie Packungen erreicht wird. So können die Kolonnen Sprüheinrichtungen aufweisen, die zum Besprühen der Kolonneneinbauten ausgelegt sind. Weiterhin können die Kolonneneinbauten untereinander so verbunden sein, dass kaum oder besser keine Toträume entstehen. Hierzu können die Kolonneneinbauten untereinander oder mit der Kolonne über unterbrochene Haftnähte verbunden sein. Derartige Haftnähte weisen mindestens etwa 2, vorzugsweise mindestens etwa 5 und besonders bevorzugt mindestens etwa 10 Unterbrechungen auf 1 m Haftnahtlänge auf. Die Länge dieser Unterbrechungen kann so gewählt sein, dass diese mindestens etwa 10, vorzugsweise mindestens etwa 20 und besonders bevorzugt mindestens etwa 50 %, im allgemeinen aber nicht mehr als 95 % der Haftnahtlänge ausmachen. Eine andere bauliche Maßnahme kann darin liegen, dass in den Kolonneninnenbereichen, insbesondere die mit der Methacrylsäureester in Kontakt kommen, weniger als etwa 50 %, vorzugsweise weniger als etwa 25 % und besonders bevorzugt weniger als etwa 10 % aller Flächen, insbesondere von Kolonneneinbauten, horizontal verlaufen. So können beispielsweise die in das Innere der Kolonne mündenden Stutzen konisch bzw. mit schrägen Flächen ausgestaltet sein. Ferner kann eine Maßnahme darin bestehen, die während des Betriebs der Kolonne im Kolonnensumpf befindliche Menge flüssiger von Methacrylsäureester so gering wie möglich zu halten und anderseits eine Überhitzung dieser Menge trotz moderaten Temperaturen und großen Verdampfungsflächen während des Verdampfen zu vermeiden. Hierbei kann es vorteilhaft sein, dass die Flüssigkeitsmenge im Kolonnesumpf im Bereich von etwa 0,1 bis 15 % und vorzugsweise etwa 1 bis 10 % der Gesamtmenge an Methacrylsäureester in der Kolonne ausmacht. Die in diesem Abschnitt vorgeschlagenen Maßnahmen können auch bei der Destillation von Methacrylsäure Anwendung finden.

Im Rahmen der Reinigung des Methacrylsäureesters werden dessen hochsiedende Bestandteile durch Destillation vom Produkt getrennt. Hierzu wird der Kolonnensumpf mit Dampf beheizt. Die Sumpftemperatur beträgt dabei vorzugsweise etwa 50 bis etwa 80 °C, insbesondere etwa 60 bis etwa 75 °C bei Wandtemperatur von weniger als etwa 120 °C.

Das im Kolonnensumpf anfallende Material wird vorzugsweise kontinuierlich abgeführt und über einen Wärmetauscher oder eine Kaskade von mehreren Wärmetauschern auf eine Temperatur in einem Bereich von etwa 40 bis etwa 80°C, vorzugsweise etwa 40 bis etwa 60°C und besonders bevorzugt in einem Bereich von etwa 50 bis 60°C abgekühlt.

Dieses Material, das überwiegend Methacrylsäureester, Hydroxyisobuttersäureester, Methacrylsäure und Stabilisatorkomponenten enthält, wird anschließend über einen Lagerbehälter, beispielsweise entsorgt oder einer anderweitigen Verwendung zugeführt. Es hat sich in vielen Fällen als vorteilhaft erwiesen, wenn das im Kolonnensumpf gewonnene Material in die Veresterungsreaktion zurückgeführt wird. Beispielsweise wird dabei das Material aus dem Kolonnensumpf in den ersten Veresterungskessel rückgeführt. Daraus ergibt sich der Vorteil, dass im Hinblick auf eine möglichst wirtschaftliche Fahrweise und eine möglichst hohe Ausbeute im Kolonnensumpf enthaltene, höhersiedende Verbindungen in die Veresterungsreaktion zurückgeführt werden.

Am Kolonnenkopf wird der destillativ aufgereinigte Methacrylsäureester entnommen und über einen Wärmetauscher oder eine Kaskade von zwei oder mehr Wärmetauschern gekühlt. Dabei kann die Wärme der Brüden durch wassergekühlte Wärmetauscher oder durch solegekühlte Wärmetauscher oder durch eine Kombination aus beidem abgeführt werden. Es hat sich in einigen Fällen bewährt, wenn die Brüden aus der Destillationskolonne in zwei oder mehr parallel geschaltete Wärmetauscher überführt werden, die mittels Wasserkühlung betrieben werden. Die nicht kondensierten Anteile aus den wassergekühlten Wärmetauschern können beispielsweise in einen solegekühlten Wärmetauscher oder eine Kaskade von zwei oder mehr solegekühlten Wärmetauschern eingeleitet werden, die seriell oder parallel angeordnet sein können. Die aus den Wärmetauschern erhältlichen Kondensate werden in einen Sammelbehälter eingeleitet und mittels einer Pumpe über einen weiteren Wärmetauscher oder eine Kaskade von zwei oder mehr weiteren Wärmetauschern einem Pufferbehälter zugeführt. Der Kondensatstrom wird dabei beispielsweise über eine Kaskade von einem oder zwei wassergekühlten Wärmetauschern und einem oder zwei solegekühlten Wärmetauschern auf eine Temperatur in einem Bereich von etwa 0 bis etwa 20°C, vorzugsweise etwa 0 bis etwa 15°C und besonders bevorzugt in einem Bereich von etwa 2 bis 10 °C heruntergekühlt.

Dem Kondensatstrom wird ein Teilstrom entnommen, der über den Kolonnenkopf in die Destillationskolonne zurückgeführt wird. Die Einspeisung des Kondensatstromes in den Kolonnenkopf kann dabei grundsätzlich auf beliebige Weise z.B. über Verteiler erfolgen. Es kann jedoch vorteilhaft sein, wenn ein Teil des Kondensatstromes oberhalb des Kolonnenkopfes in die Brüdenleitung eingespeist, beispielsweise eingesprüht, wird. Weiterhin ist es bevorzugt, dass mit dieser Einspeisung Stabilisator in den Kolonnenkopf eingetragen wird.

Ein weiterer Teilstrom des zur Rückführung in die Kolonne vorgesehenen Kondensates kann beispielsweise vor Einbringung in die Brüdenleitung abgezweigt und direkt in den Kolonnenkopf eingebracht werden. Auch hier ist es bevorzugt, dass mit dieser Einspeisung Stabilisator in den Kolonnenkopf eingetragen wird. Die Einbringung in den Kolonnenkopf kann dabei beispielsweise dergestalt geschehen, dass das Innere des Kolonnenkopfes mit dem Kondensat so besprüht wird, dass sich keine Ruhezonen im Kolonnenkopf ausbilden können, an denen eine Polymerisation des Methacrylsäureesters erfolgen kann. Es kann darüber hinaus vorteilhaft sein, wenn einem Kondensatteilstrom, der in die Kolonne zurückgeführt wird, ein Stabilisator zur Verhinderung von Polymerisation zugefügt wird. Dies kann beispielsweise dadurch geschehen, dass dem zum Besprühen des Kolonnenkopfes vorgesehenen Kondensatteilstrom eine entsprechende Menge an Polymerisationsinhibitor als Stabilisator zugefügt wird. Dabei hat es sich in einigen Fällen als vorteilhaft erwiesen, wenn der Kondensatteilstrom nach der Zugabe des Stabilisators, aber vor dem Eintritt in den Kolonnenkopf eine geeignete Mischvorrichtung, vorzugsweise einen Statikmischer durchläuft, um eine möglichst uniforme Verteilung des Stabilisators im Kondensatteilstrom zu erzielen.

Die im Rahmen des Reinigungsverfahrens anfallenden, nicht kondensierbaren gasförmigen Stoffe werden beispielsweise der Entsorgung zugeführt.

Das im Pufferbehälter befindliche Rohprodukt wird mit Hilfe eines Solekühlers auf einer Temperatur von etwa 0 bis etwa 20°C, vorzugsweise etwa 0 bis etwa 15 °C und besonders bevorzugt in einem Bereich von etwa 2 bis 10°C gehalten.

Um ggf. weitere Verunreinigungen aus dem Produkt zu entfernen und zur Reinstmethacrylsäurealkylestern zu gelangen, kann das Produkt noch einer adsorptiven Reinigungsstufe unterzogen werden. Es hat sich dabei beispielsweise bewährt, wenn das Reinprodukt ganz oder zumindest ein Teil des Reinproduktes mit Hilfe eines Molekularsiebes weiter gereinigt wird. Besonders saure Verunreinigungen, insbesondere im Rahmen des Herstellungsverfahrens gebildete Ameisensäure, kann so auf einfache Weise aus dem Produktstrom entfernt werden. Dabei hat es sich darüber hinaus in einigen Fällen bewährt, wenn der Produktstrom nach dem Durchlaufen der adsorptiven Reinigungsstufe noch einen oder mehrere Filter durchläuft, um ggf. im Produkt enthaltene Feststoffe zu entfernen.

Die im Rahmen der Aufarbeitung anfallenden Stoffströme umfassen überwiegend polymerisierbare Verbindungen. Um, wie bereits im Rahmen dieses Textes mehrfach beschrieben, die Ausbildung von Ruhezonen zu unterbinden, hat es sich auch im Fall des hier beschriebenen Verfahrens als vorteilhaft herausgestellt, wenn die Teile der Anlage, die mit Methacrylsäureester in Berührung kommen, ständig mit Methacrylsäureester überströmt sind. Im Rahmen einer weiteren Ausführungsform des hier vorgestellten Verfahrens wird daher ein Teilstrom an Methacrylsäureester nach dem Pufferbehälter, jedoch vor der adsorptiven Reinigungsstufe entnommen, um die Kopfbereiche derjenigen Wärmetauscher zu überspülen, welche die aus der Destillationskolonne stammenden Brüden aufnehmen.

Das im Rahmen der Reinigungsstufe gewonnene Produkt wird anschließend mit einer Temperatur in einem Bereich von etwa -5 bis etwa 20°C, vorzugsweise etwa 0 bis etwa 15°C und besonders bevorzugt in einem Bereich von etwa 2 bis 10°C der Reinigungsstufe entnommen.

### Strippen der verbrauchten Säure

Im Rahmen des hier vorgestellten Verfahrens kann es beispielsweise in einem weiteren Verfahrenselement sinnvoll sein, die bei dem Verfahren anfallende, verbrauchte Schwefelsäure einer Reinigung zu unterziehen, um sie anschließend wieder in das Verfahren zurückzuführen. Dabei kann beispielsweise ein Strom mit verbrauchter Schwefelsäure, wie er sich aus der Veresterung erhalten lässt, in einem Flotationsbehälter mit Dampf beaufschlagt werden. Dabei kann sich zumindest ein Teil der enthaltenen Feststoffe auf der Oberfläche der Flüssigkeit abscheiden, wobei diese abgeschiedenen Feststoffe ausgekreist werden können. Die Brüden werden anschließend in einem Wärmetauscher, vorzugsweise mit Wasserkühlung, kondensiert, gekühlt und in die Veresterungsreaktion zurückgeführt.

Es hat sich dabei als in einigen Fällen als vorteilhaft erwiesen, wenn zur Verminderung von Korrosion in den Wärmetauschern und zur weiteren Verbesserung der Kühlwirkung ein Gemisch aus Wasser und organischen Verbindungen, wie es im Rahmen der Veresterung bei der Reinigung des hergestellten Methacrylsäureesters durch Waschen erhalten wird, in die Wärmetauscher derart eingebracht wird, dass die Köpfe der Wärmetauscher mit diesem Gemisch besprüht werden. Neben der korrosionsvermindernden Wirkung und der Kühlung der Säure im Wärmetauscher weist diese Vorgehensweise einen weiteren Vorteil auf. Material, das aus der Veresterung stammt (ein Gemisch aus Wasser und überwiegend Methanol), wird zusammen mit der aus eben diesem Prozess stammenden Methacrylsäure und Methacrylsäureester in den Veresterungsprozess zurückgeführt. Im Stripper werden durch die oben beschriebene Flotation Gemische von Säure und Feststoffen erhalten. Diese werden nach ihrer Abtrennung einer beliebigen weiteren Verwendung oder der Entsorgung zugeführt. Möglich ist beispielsweise, das erhaltene Gemisch in einer Spaltanlage zu verbrennen und damit wieder Schwefelsäure zu erzeugen und um einen Teil der im Prozess eingesetzten Energie zurückzugewinnen.

Die beim Strippen anfallenden, nicht kondensierbaren gasförmigen Verbindungen werden einer beliebigen Weiterverwendung zugeführt oder entsorgt.

Die hier beschriebene Anlage zur Entfernung von Feststoffen aus der verbrauchten Säure sowie zur Rückführung von Material aus dem Veresterungsprozess in eben diesen Prozess kann aus Gründen der Betriebssicherheit beispielsweise auch doppelt ausgeführt sein. So können die zwei oder mehr Flotationsbehälter zeitlich versetzt eingesetzt werden. Da sich in diesen Behältern Feststoffe absetzen können, ist es vorteilhaft, diese zu entfernen, wenn der jeweilige Flotationsbehälter sich nicht im Einsatz befindet.

Das Vorstehende wird nunmehr anhand von nicht limitierenden Zeichnungen und Beispielen näher erläutert. Es zeigen schematisch:
- Fig. 1:: einen Anlagenverbund zur Herstellung und Verarbeitung von mit Methacrylsäure bzw. Methylmethacrylat,
- Fig. 2:: eine Anlage zur Herstellung von Acetoncyanhydrin,
- Fig. 3:: eine Aufarbeitungsanlage von Acetoncyanhydrin,
- Fig. 4:: eine Amidierungsanlage,
- Fig. 5:: eine Veresterungsanlage,
- Fig. 6:: eine Anlage zur Vorreinigung des Esters,
- Fig. 7:: eine Feinreinigungsanlage des Esters,

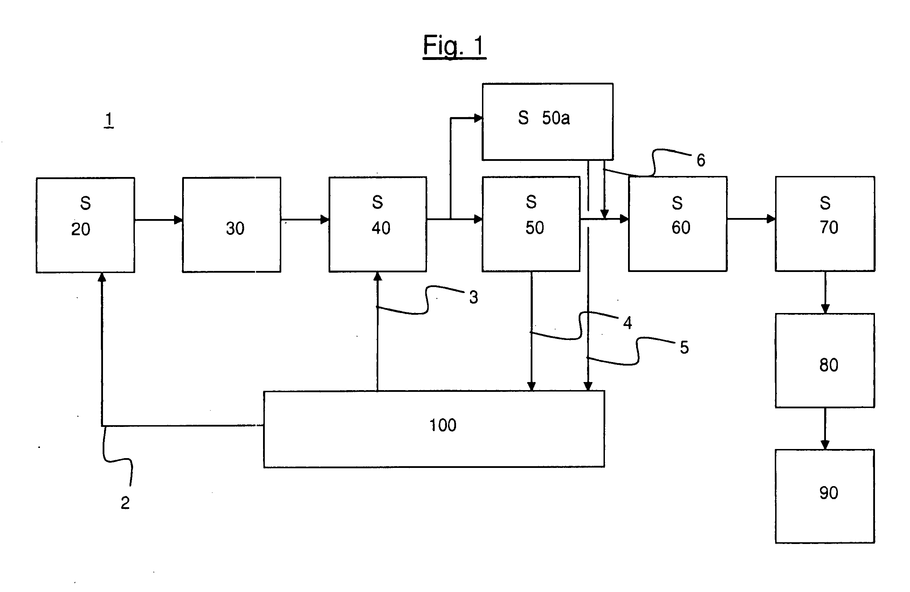

In Fig. 1 sind die bevorzugten Elemente einen Anlagenverbund 1 zur Herstellung von Methacrylsäure bzw. Methacrylsäureestern und deren Weiterverarbeitungsprodukten gezeigt. Der Anlagenverbund 1 weist verschiedene untereinander meist fluidleitenden verbundene Anlagen als Elemente dieses Verbunds auf. Zu diesem Anlagenverbund gehört die Acetoncyanhydrinherstellung 20, gefolgt von der Acetoncyanhydrinaufarbeitung 30, gefolgt von einer Amidierung 40, gefolgt von einer Veresterung/Hydrolyse 50/50a), gefolgt von einer Aufarbeitung für Ester oder Methacrylsäure 60 wiederum gefolgt von einer Feinreinigung 70, nach der der Ester, meist Methylmethacrylat, oder Methacrylsäure vorliegt. Der so gewonnene Reinester/Reinsäure kann einer Weiterverarbeitungsanlage 80 zugeführt werden. Als Weiterverarbeitungsanlagen 80 kommen vor allem Polymerisationsvorrichtungen und Reaktoren für weitere organische Reaktionen in Betracht. In den Polymerisationsreaktoren können Polymethacrylate hergestellt werden und in den Reaktoren für organische Reaktionen können die hier gewonnenen reinen Monomere zu weiteren organischen Verbindungen umgesetzt werden. Auf die Weiterverarbeitungsanlage oder die Weiterverarbeitungsanlagen 80 folgt eine Konfektionierung 90. Sofern es sich bei den Weiterverarbeitungsprodukten um Polymere aus der Methacrylsäure oder Methacrylsäureester, insbesondere Methylmethacrylat, handelt werden diese zu Fasern, Formmassen, insbesondere Granulat, Folien, Platten, Automobilteile und anderen Formkörpern durch geeignete Geräte wie Extruder, Blasextruder, Spritzgussgeräte, Spinndüsen und dergleichen, weiterverarbeitet. Weiterhin beinhaltet der Anlagenverbund 1 in vielen Fällen eine Schwefelsäureanlage 100. Hierbei kommen grundsätzlich alle dem Fachmann hierfür geeignet erscheinenden Schwefelsäureanlagen in Betracht. Beispielsweise sei auf die in Kapitel 4, Seite 89 ff. in "Integrated Pollution Prevention and Control -Draft Reference Document on Best Avalible Techniques for the Manufacture of Large Volume Inorganic Chemicals - Amonia Acids and Fertelizers", erhältlich über die Europäische Kommission, in diesem Zusammenhang verwiesen. Die Schwefelsäureanlage 100 ist mit einer Reihe anderer Anlagen verbunden. So wird die Acetoncyanhydrinherstellung 20 über eine Schwefelsäureleitung 2 mit konzentrierter Schwefelsäure versorgt. Außerdem besteht eine weitere Schwefelsäureleitung 3 zwischen der Schwefelsäureanlage 100 und der Amidierung 40. Die auch als "Spent Acid" bezeichnete verdünnte Schwefelsäure aus der Veresterung 50 (Hydrolyse 50a) wird zu der Schwefelsäureanlage 100 durch die Leitungen für verbrauchte Schwefelsäure 4 bzw. 5 überführt. In der Schwefelsäureanlage 100 kann die verdünnte Schwefelsäure aufgearbeitet werden. Die Aufarbeitung der verdünnten Schwefelsäure kann beispielsweise wie in WO 02/23088 A1 oder WO 02/23089 A1 beschrieben erfolgen. Im Allgemeinen sind die Anlagen aus dem Fachmann geläufigen und für die jeweiligen Beanspruchungen geeignet erscheinenden Materialien ausgeführt. Meist handelt es sich hierbei um Edelstahl, der insbesondere eine besondere Säurefestigkeit besitzen muss. Die Bereiche der Anlagen, die mit Schwefelsäure und insbesondere mit konzentrierter Schwefelsäure betrieben werden, sind darüber hinaus mit keramischen Materialien oder Kunststoffen ausgekleidet und geschützt. Zudem kann die in der Methacrylsäureanlage 50a erhaltene Methacrylsäure über eine Methacrylsäureleitung 6 der Vorreinigung 60 zugeführt werden. Weiterhin hat es sich bewährt, dass in der Acetoncyanhydrinherstellung 20, der Amidierung 40, der Veresterung 50, der Hydrolyse 50a, der Vorreinigung 60 und auch der Endreinigung 70 einen mit "S" gekennzeichneten Stabilisators zuzusetzen.

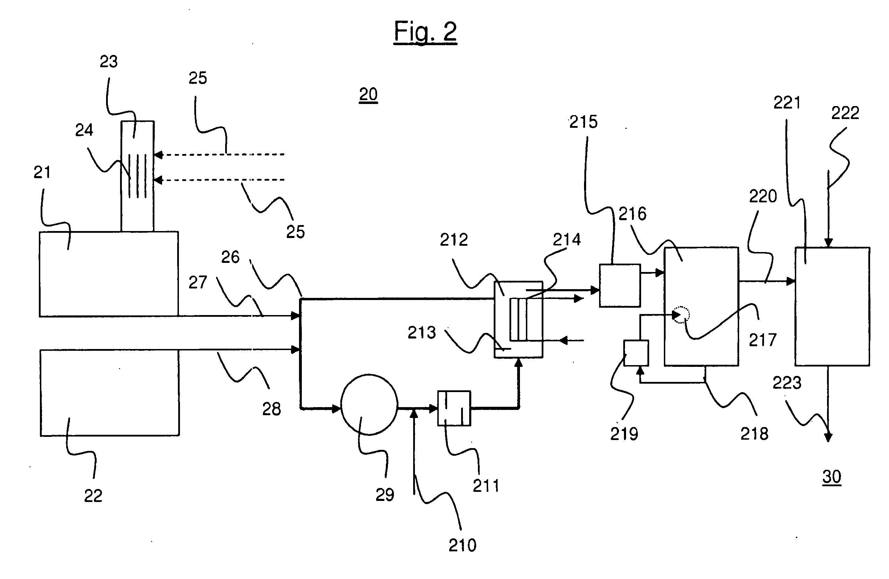

In der in Figur 2 dargestellten Acetoncyanhydrinherstellung 20 wird das Aceton in einem Acetonbehälter 21 und die Blausäure in einem Blausäurebehälter 22 bereitgestellt. Der Acetonbehälter 21 weist einen Waschturm 23 auf, der in seinem oberen Bereich einen oder mehrere Kühlelemente 24 aufweist. In den Waschturm 23 mündet eine Reihe von Abgasleitungen 25, die aus verschiedenen Anlagen des Anlagenverbunds 1 stammen. In einen Schlaufenreaktor 26 wird das Aceton über die Acetonzuleitung 27 und die Blausäure über die Blausäurezuleitung 28 eingespeist stromab der Blausäurezuleitung 28 befindet sich eine Pumpe 29, wiederum gefolgt von einer Katalysatoreinspeisung 210, auf die ein statischer Mischer 211 folgt. Hiernach schließt sich ein Wärmetauscher 212 an, der eine Reihe von Strömungswiderständen 213 und mindestens eine Kühlleitung 214 aufweist. In dem Schlaufenreaktor 26 wird das aus Aceton, Blausäure und Katalysator bestehende Reaktionsgemisch zu einem erheblichen Teil in einem Kreislauf gefahren, der durch fette Linien gekennzeichnet ist. Aus dem Wärmetauscher 212 wird die Reaktionsmischung über die Strömungswiderstände entlang der Kühlleitungen 214 geführt und ein Teil des Kreislaufstromes in einen weiteren Wärmetauscher 215 geleitet, an den sich ein Sammelbehälter 216 anschließt, in dem sich eine Düse 217 als Teil eines Kühlkreislaufs 218 mit einem Wärmetauscher 219 befindet, wodurch das Reaktionsprodukt zum einen in Bewegung und zum anderen kühl gehalten wird. Über eine sich an den Sammelbehälter 216 anschließende Ableitung 220 ist ein Stabilisierbehälter 221 angeschlossen, an den eine Schwefelsäurezuleitung 222 mündet und aus dem das Rohacetoncyanhydrin durch die Ableitung 223 in die Acetoncyanhydrinaufarbeitung 30 geführt wird.

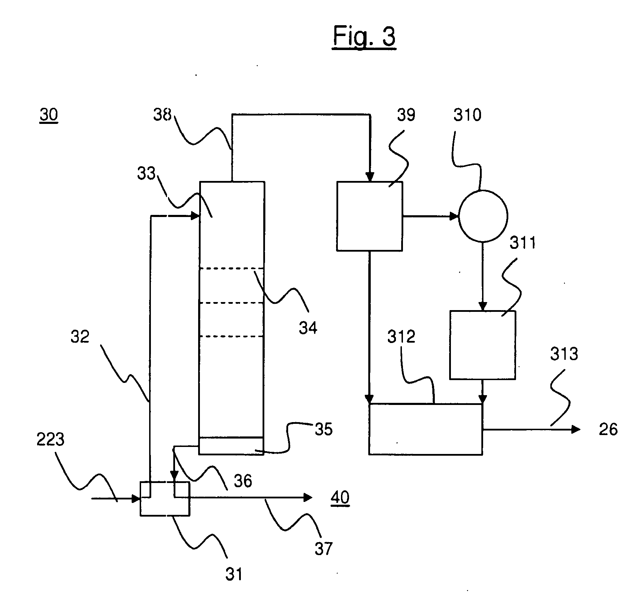

In Figur 3 mündet aus der Cyanhydrinherstellung 20 kommend die Ableitung 223 in einen Wärmetauscher 31, in dem der aus der Cyanherstellung 20 kommende Strom erwärmt wird. An den Wärmetauscher 31 schließt sich eine Brüdenzuleitung 32 an, die in dem oberen, vorzugsweise dem Kopfbereich einer Kolonne 33 mündet. Die Kolonne 33 weist eine Vielzahl von Packungen 34 auf, die meist als Böden ausgestaltet sind. Im unteren Bereich der Kolonne 33 befindet sich der Kolonnensumpf 35 aus dem eine Sumpfableitung 36 in den Wärmeaustauscher 31 führt und den über die Ableitung 233 in den Wärmeaustausch 31 geführten Ströme erwärmt. An den Wärmetauscher 31 schließt sich eine Reinproduktführung 37 an, auf die die Amidierung 40 stromab folgt. Im Kopfbereich der Kolonne 33 befindet sich eine Kopfableitung 38, die in einen Wärmetauscher 39 mündet, an den sich eine Vakuumpumpe 310 anschließt, die wiederum in einen Wärmetauscher 311 mündet. Sowohl der Wärmetauscher 39 als auch der Wärmetauscher 311 sind über Leitungen mit einem Kühlbehälter 312 verbunden an den sich eine Rückführung 313 anschließt, die mit dem Schlaufenreaktor 26 in der Acetoncyanhydrinherstellung 20 verbunden ist.

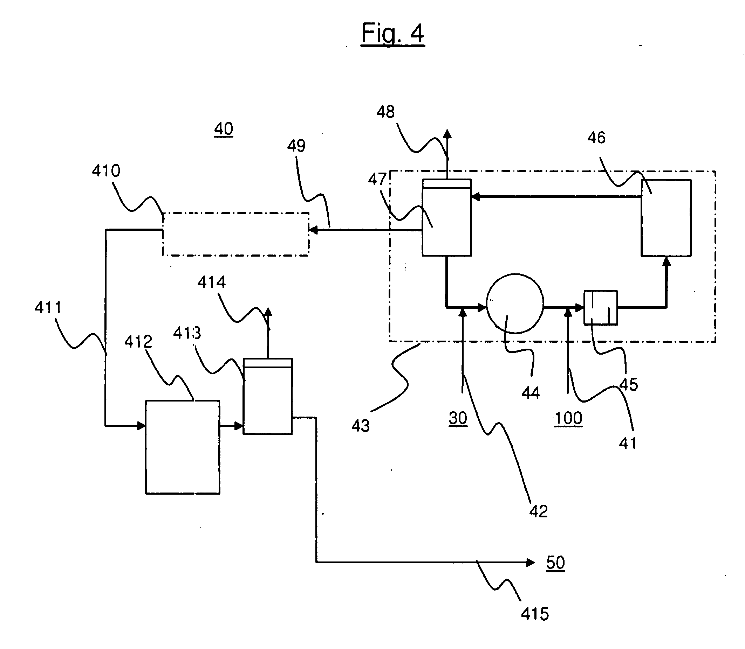

Die in Fig. 4 abgebildete Amidierung 40 weist zunächst eine Acetoncyanhydrinzuführung 41 und eine Schwefelsäurezuführung 42 auf, die in einen Schlaufenreaktor 43 münden. Die mit der Acetoncyanhydrinaufarbeitung 30 verbundene Acetoncyanhydrinzuführung 41 mündet in den Kreislauf des Schlaufenreaktors 43 nach einer Pumpe 44 vor einem Mischer 45. Vor dieser Pumpe 44 mündet die Schwefelsäurezuführung 42. Stromab des Mischers 45 folgt ein Wärmeaustauscher 46 der wiederum in einen Gasabscheider 47 mündet aus dem zum einen eine Gasableitung 48 und eine Zuleitung 49 zu einem weiteren Schlaufenreaktor 410 abgeht. Der weitere Schlaufenreaktor 410 oder ein Dritter ist vergleichbar wie der erste Schlaufenreaktor 43 aufgebaut. Von dem weiteren Schlaufenreaktor 410 geht eine Zuleitung 411 in einen Wärmeaustauscher 412, auf den ein Gasabscheider 413 folgt, von dem zu einem eine Gasableitung 414 und eine Amidleitung 415 abgeht, die zu der Veresterung/Verseifung 50 /MAS-Anlage 50a führt.

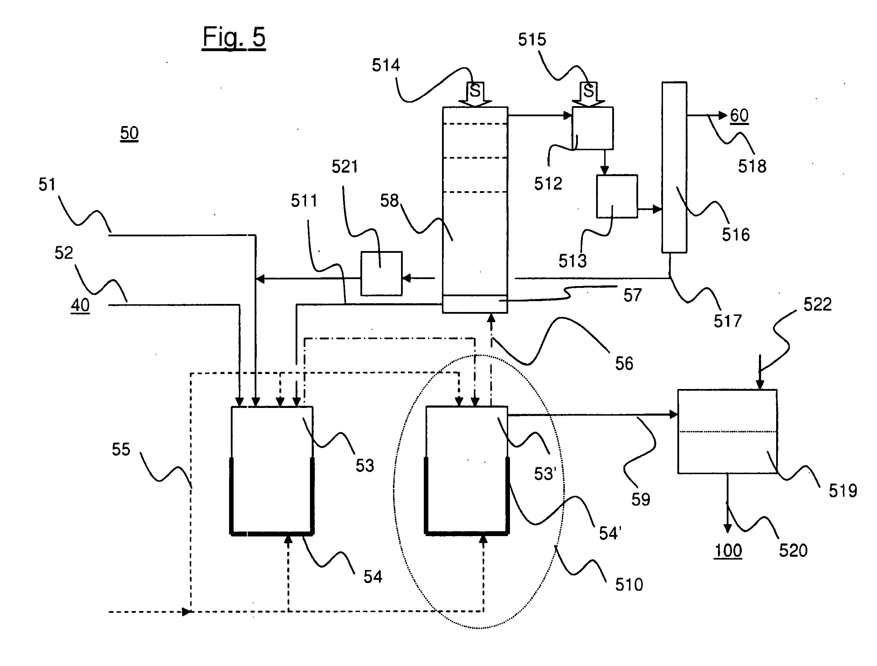

Die Figur 5 zeigt die Veresterung 50, in der eine Wasser und organische Lösungsmittel führende Lösungsmittelleitung 51 und eine mit der Amidierung 40 verbundene Amidleitung 52 in einen Kessel 53 münden, der durch eine Kesselheizung 54 beheizbar ist. In den Kessel 53 mündet weiterhin eine gestrichelt gezeichnete Alkoholleitung 55. Die Alkoholleitung 55 mündet sowohl in dem oberen als auch in dem unteren Bereich des Kessels 53. Über eine mit Strichpunkt gekennzeichnete Esterbrüdenleitung 56 ist der erste Kessel 53 mit einem weiteren Kessel 53' verbunden, der eine weitere Kesselheizung 54' aufweist. Auch dieser weitere Kessel 53' ist sowohl von unten als auch von oben mit der Alkoholleitung 55 verbunden. An den oberen Bereich des Kessels 53' schließt sich die Esterbrüdenleitung 56 an, die in einen Sumpf 57 einer Kolonne 58 mündet. Weiterhin befindet sich im oberen Bereich des Kessels 53' eine Leitung für verdünnte Schwefelsäure 59. Eine in der gepunkteten Ellipse eingerahmte Kesseleinheit 510 wird aus einem beheizbaren Kessel 53 und 54 mit Alkoholleitung 55 und Esterbrüdenleitung 56 gebildet. Es können ein, zwei oder mehr derartiger Kesseleinheiten kaskadenartig aufeinander folgen, wobei jede dieser Kesseleinheiten 510 über die Esterbrüdenleitung 56 mit dem Sumpf 57 der Kolonne 58 verbunden ist. Aus dem Sumpf 57 der Kolonne 58 führt weiterhin eine Schwersiederleitung 511 zum Kessel 53, um Wasser und organische Lösungsmittel wieder der Veresterung zuzuführen. Im Oberbereich, vorzugsweise dem Kopf, der Kolonne 58, schließt sich über eine geeignete Leitung ein erster Wärmetauscher 512 gefolgt von einem weiteren Phasentrenner 513 an. Sowohl am Kopf der Kolonne 58 als auch in dem ersten Wärmetauscher 512 können eine erste Stabilisatorzuführung 514 (Stabilisator mit "S" gekennzeichnet) sowie eine weitere Stabilisatorzuführung 515 vorgesehen sein, um einen eine unerwünschte Polymerisation verhindernden Inhibitor bzw. Stabilisator zuzuführen. An den weiteren Phasentrenner 513 schließt sich ein Wäscher 516 an, in dessen unteren Bereich eine Lösemittelleitung 517 abgeht, die über einen Wärmetauscher 521 in der Lösungsmittelleitung 51 mündet. Von dem oberen Bereich des Wäschers 516 geht eine Rohesterleitung ab, die in die Esteraufarbeitung 60 mündet. Die aus dem oberen Bereich des Kessels 53' bzw. des Kessels der letzten Kesseleinheit 510 abgehende Spendsäureleitung 59 mündet in einen Flotationsbehälter 519 zum Abtrennen der Feststoffe bzw. in der Spendsäure nicht löslichen Bestandteile. Aus dem Flotationsbehälter 519 geht eine Spentsäureableitung 520 in die Schwefelsäureanlage 100 sowie eine, die niedriegsiedenden Bestandteile führende, Niedersiederbrüdenleitung 522 zur weiteren Aufarbeitung und Rückführung in die Veresterung.

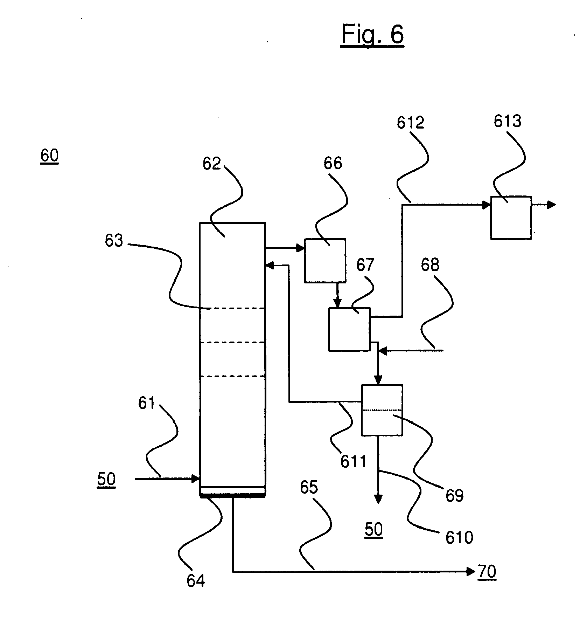

Die in Figur 6 gezeigt Esteraufarbeitung schließt sich über eine Rohesterleitung 61 an die Veresterung 50 an, wobei die Rohesterzuleitung 61 in den mittleren Bereich einer Vakuumdestillationskolonne 62 mündet. Diese Kolonne 62 weist Kolonneneinbauten 63 und eine im unteren Bereich der Kolonne 62 angeordnete Sumpfheizung 64 auf. Von dem unteren Bereich der Kolonne 62, der den Sumpf dieser Kolonne darstellt, geht eine Esterableitung 65 ab, die in die Esterfeinreinigung 70 mündet und den von Leichtsiedern befreiten Rohester somit der Feinreinigung zuführt. Im oberen Bereich der Kolonne 62, meist im Kopf, schließt sich über eine Ableitung ein erster Wärmetauscher 66 und ein weiterer oder mehrere Wärmetauscher 67 an, auf den ein Phasentrenner 69 folgt. In dem Phasentrenner 69 wird der Strom 68 und die aus dem Wärmetauscher 67 stammende Mischung in organische und wässrige Bestandteile aufgeteilt, wobei sich an den Phasentrenner 69 eine Rückführung 611 im oberen Bereich anschließt, die im oberen Bereich der Kolonne 62 einmündet. Im unteren Bereich des Abscheiders befindet sich eine Wasserableitung 610, die in die Veresterung 50 mündet, um das abgetrennte Wasser der Veresterung wieder zuzuführen. An die Wärmetauscher 66 und 67 schließt sich über eine Unterdruckleitung 612 eine Unterdruckerzeuger 613 an.

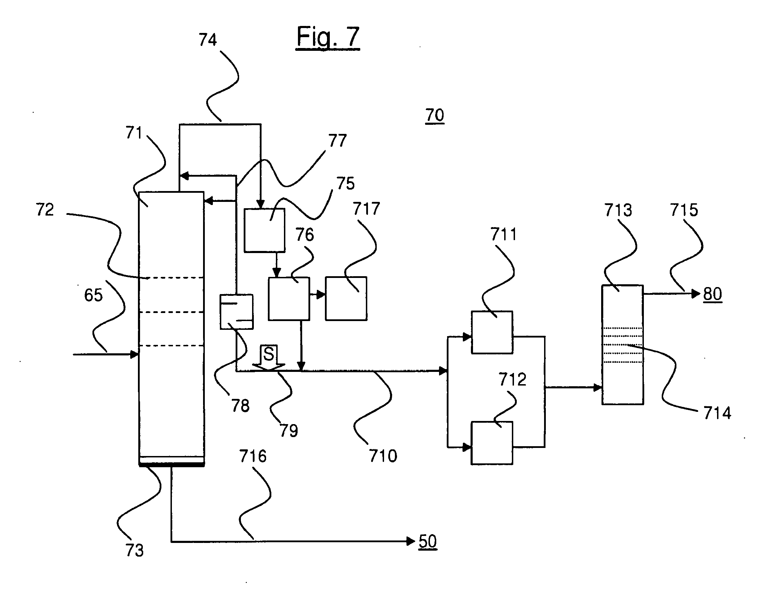

In Figur 7 mündet die aus der Esteraufarbeitung 60 stammende Esterableitung 65 in eine Destillationskolonne 71. Diese umfasst mehrere Kolonneneinbauten 71 sowie im unteren Bereich der Destillationskolonne 71 eine Kolonnensumpfheizung 73. Vom Kopfbereich der Destillationskolonne 71 geht eine Reinesterbrüdenleitung 74 in einen ersten Wärmetauscher 75, auf den ein (oder mehrere) weiterer Wärmetauscher 76 folgen, die mit einem Unterdruckerzeuger 717 verbunden sind. Der Ausgang des weiteren Wärmetauschers 76 weist eine Leitung auf, von der zum einen eine Esterrückführung 77 in den oberen Bereich der bzw. in den Kopf der Destillationskolonne 71 mündet. Die Esterrückführung 77 weist eine Stabilisatorzudosierung 79 auf, die in der Esterrückführung 77 vor einem Mischer 78 angeordnet ist. Zum anderen geht von der Leitung des weiteren Wärmetauschers 76 eine Reinesterableitung 710 ab. An diese schließt sich in Reihenschaltung ein zusätzlicher Wärmetauscher 711 und ein anderer Wärmetauscher 712 an. Auf diese folgt ein Molekularsiebbehälter 713, der Molekularsiebpackungen 714 aufweist. Durch das Molekularsieb weiter gereinigt, wird der Reinstester durch die sich an den Molekularsiebbehälter anschließende Reinstesterableitung in die Weiterverarbeitungsanlage 80 überführt.

### BEISPIELE

Ein Methylmethacrylatstrom von 10 m³/h wurde durch einen 50 Literbehälter mit einer Molsiebfüllung aus Baylith^{®}, kommerziel erhältlich über die Kurt Obermeier GmbH & Co. KG, Deutschland, von 40 kg bei 35°C geleitet. Der Strom wurde erst nach Erreichen der Aufnahmekapazität des Molekularsiebs zur Entfernung des Molekularsiebs unterbrochen. Nach dem Durchströmen des Molekularsiebs wurde am Ausgang des Behälters ein Reinst-Methylmethacrylat abgenommen, dass eine klare, farblose Konsistenz aufwies. Es wurde eine signifikante Abreicherung von Ameisensäure, Methacrylsäure in dem Reinst-Methylmethacrylat gegenüber dem Methylmethacrylatstrom durch HPLC bestimmt. Nach der Betriebszeit wurde keine Polymerablagerung auf dem Molekularsieb beobachtet. Die Polymerisation des Reinst-Methylmethacrylats ergab sowohl bei der Polymerisation als auch bei den Eigenschaften des so erhaltenen Polymethylmethacrylats keine Unterschiede zu einem durch herkömmliche Destillation gereinigten Methylmethacrylat, das unter identischen Bedingungen polymerisiert wurde.

### Bezugszeichenliste

- 1: Anlagenverbund
- 2: Schwefelsäureleitung
- 3: weitere Schwefelsäureleitung
- 4: verbrauchte Schwefelsäureleitung - Ester
- 5: verbrauchte Schwefelsäureleitung - Säure
- 6: Methacrysäureleitung
- 20: Acetoncyanhydrinherstellung
- 30: Acetoncyanahydrinaufarbeitung
- 40: Amidierung
- 50: Veresterung
- 50a: Hydrolyse
- 60: Vorreinigung
- 70: Feinreinigung
- 80: Weiterverarbeitungsanlage
- 90: Konfektionierung
- 100: Schwefelsäureanlage
- 21: Acetonbehälter
- 22: Blausäurebehälter
- 23: Waschturm
- 24: Kühlelemente
- 25: Abgasleitungen
- 26: Schlaufenreaktor
- 27: Acetonzuleitung
- 28: Blausäurezuleitung
- 29: Pumpe
- 210: Katalysatorzuleitung
- 211: Mischer
- 212: Wärmetauscher
- 213: Strömungswiderstand
- 214: Kühlleitungen
- 215: Wärmetauscher
- 216: Sammelbehälter
- 217: Düse
- 218: Kühlkreislauf
- 219: Wärmetauscher
- 220: Ableitung
- 221: Stabilisierbehälter
- 222: Schwefelsäurezuleitung
- 223: Ableitung
- 31: Wärmetauscher
- 32: Brüdenzuleitung
- 33: Kolonne
- 34: Packungen
- 35: Kolonnensumpf mit Wärmetauscher
- 36: Sumpfableitung
- 37: Reinproduktführung
- 38: Kopfableitung
- 39: Wärmetauscher
- 310: Vakuumpumpe
- 311: Wärmetauscher
- 312: Kühlbehälter
- 313: Rückführung
- 41: Acetoncyanhydrinzuführung
- 42: Schwefelsäurezuführung
- 43: Schlaufenreaktor
- 44: Pumpe
- 45: Mischer
- 46: Wärmetauscher
- 47: Gasabscheider
- 48: Gasableitung
- 49: Zuleitung
- 410: weiterer Schlaufenreaktor
- 411: Zuleitung
- 412: Wärmetauscher
- 413: Gasabscheider
- 414: Gasableitung
- 415: Amidleitung
- 51: Lösungsmittelleitung
- 52: Amidleitung
- 53: erster Kessel
- 54: erste Kesselheizung
- 53': weiterer Kessel
- 54': weitere Kesselheizung
- 55: Alkoholleitung
- 56: Esterbrüdenleitung
- 57: Kolonnensumpf
- 58: Kolonne
- 59: Spendsäureleitung
- 510: Kesseleinheit
- 511: Schwersiederleitung
- 512: Wärmetauscher
- 513: Phasentrenner
- 514: Stabilisatorzuführung
- 515: weitere Stabilisatorzuführung
- 516: Extraktionskolonne
- 517: Lösemittelleitung
- 518: Rohesterleitung
- 519: Flotationsbehälter
- 520: Spentsäureableitung
- 521: Wärmetauscher
- 522: Niedersiederbrüdenleitung
- 61: Rohesterzuleitung
- 62: Vakuumdestillationskolonne
- 63: Kolonneneinbauten
- 64: Sumpfheizung
- 65: Esterableitung
- 66: Wärmetauscher
- 67: Wärmetauscher
- 68: Wasserzufuhr
- 69: Phasentrenner
- 610: Wasserableitung
- 611: Rückführung
- 612: Unterdruckleitung
- 613: Unterdruckerzeuger
- 71: Destillationskolonne
- 72: Kolonneneinbauten
- 73: Kolonnensumpfheizung
- 74: Reinesterbrüdenleitung
- 75: erster Wärmetauscher
- 76: weitere Wärmetauscher
- 77: Esterrückführung
- 78: Mischer
- 79: Stabilisatorzudosierung
- 710: Reinesterableitung
- 711: zusätzlicher Wärmetauscher
- 712: andere Wärmetauscher
- 713: Molekularsiebbehälter
- 714: Molekularsiebpackungen
- 715: Reinstesterableitung
- 716: Schwersiederleitung
- 717: Unterdruckerzeuger

## Patentansprüche

1. Ein Verfahren zur Aufreinigung eines Methacrylsäurealkylesters von Ameisensäure und/oder Methacrylsäure und/oder Schwefelsäure beinhaltend die Schritte:
i. Bereitstellen eines Methacrylsäurealkylesters verunreinigt mit Ameisensäure und/oder Methacrylsäure und/oder Schwefelsäure ;
ii. Adsorption mindestens eines Teils der Ameisensäure und/oder Methacrylsäure und/oder Schwefelsäure an einen Reinigungsfeststoff enthaltend mindestens 10% einer Silicium-Sauerstoff-Verbindung unter Erhalt eines Reinst-Methacrylsäurealkylesters.

2. Verfahren nach Anspruch 1, wobei Methacrylsäurealkylester vor Schritt ii. zur Vorreinigung destilliert wurde.

3. Verfahren nach Anspruch 1 oder 2, wobei der in Schritt i. bereitgestellte Methacrylsäurealkylester mindestens einer der nachfolgenden Verunreinigungen beinhaltet:
a. Ameisensäure in einer Menge von maximal 0,05 Gew.-%;
b. Methacrylsäure in einer Menge von maximal 0,1 Gew.-%;
c. Schwefeldioxid in einer Menge von maximal 0,001 Gew.-%.

4. Verfahren nach eine der vorhergehenden Ansprüche, zusätzlich beinhaltend als Schritte die thermische Regenerierung des Reinigungsfeststoffes, gefolgt von der Wiederverwendung des regenerierten Reinigungsfeststoffs in Schritt ii.

5. Verfahren nach einem der vorhergehenden Ansprüche, wobei mindestens Schritt ii. kontinuierlich erfolgt.

6. Ein Verfahren zur Herstellung eines Reinst-Methacrylsäurealkylesters, beinhaltend als Schritte
- Erzeugen eines gegebenenfalls vorgereinigten Methacrylsäurealkylesters;
- Reinigen des Methacrylsäurealkylesters durch ein Verfahren nach einem der vorhergehenden Ansprüche.

7. Verfahren nach Anspruch 6, wobei das Erzeugen des Methacrylsäurealkylesters die Schritte beinhaltet
i. Bereitstellen eines Acetoncyanhydrins;
ii. in Kontakt bringen des Acetoncyanhydrins mit einer anorganischen Säure unter Erhalt eines Methacrylamids;
iii. in Kontakt bringen des Methacrylamids mit einem Alkohol unter Erhalt eines Methacrylsäurealkylesters;
iv. gegebenenfalls vorreinigen des Methacrylsäurealkylesters.

8. Vorrichtung zur Herstellung von Methacrylsäurealkylestern, beinhaltend fluidleitend miteinander verbunden
- ein Anlagenelement zur Herstellung von Acetoncyanhydrin, gefolgt von;
- einem Anlagenelement zur Herstellung von Methacrylamid, gefolgt von;
- einem Anlagenelement zur Herstellung von Methacrylsäurealkylester, gefolgt von;
- einem Anlagenelement zur Reinigung des Methacrylsäurealkylesters, gefolgt von;
- einem Anlagenelement zur Polymerisation, gefolgt von;
- einem Anlagenelement zur Konfektionierung,
wobei das Anlagenelement zur Reinigung einen Behälter mit einem Reinigungsfeststoff enthaltend mindestens 10% einer Silicium-Sauerstoff-Verbindung beinhaltet.

9. Verfahren nach einem der Ansprüche 1 bis 7 oder Vorrichtung nach Anspruch 8, wobei eine Vielzahl von Teilchen des Reinigungsfeststoffs in einem Reinigungsbehälter aufgenommen sind, wobei der Reinigungsbehälter von flüssigen Methacrylsäurealkylester durchströmbar ist.

10. Verfahren oder Vorrichtung nach einem der vorhergehenden Ansprüche, wobei der Reinigungsfeststoff eine spezifische Oberfläche in einem Bereich von 400 bis 1500 m²/g aufweist.

11. Verfahren oder Vorrichtung nach einem der vorhergehenden Ansprüche, wobei der Reinigungsfeststoff eine nach DIN 66135-1 bestimmte Porengröße im Bereich von 0,01 bis 2 nm aufweist.

12. Verfahren oder Vorrichtung nach einem der vorhergehenden Ansprüche, wobei der Reinigungsfeststoff eine Schüttdichte im Bereich von 920 bis 960 mg/cm³ aufweist.

13. Verfahren oder Vorrichtung nach einem der vorhergehenden Ansprüche, wobei mindestens 80 Gew.-% der Teilchen des Reinigungsfeststoffs eine Teilchengröße im Bereich von 0,5 bis 10 mm aufweisen.

14. Verfahren oder Vorrichtung nach einem der vorhergehenden Ansprüche, wobei die Gewichtsmittel der Teilchengröße in einem Bereich von 0,5 bis 10 liegt.

15. Verfahren oder Vorrichtung nach einem der vorhergehenden Ansprüche, wobei der Reinigungsfeststoff mindestens 10 Gew.-% eines Zeoliths beinhaltet.

16. Verfahren nach Anspruch 6 oder 7, wobei das Verfahren in einer Vorrichtung nach einem der Ansprüche 8 bis 15 erfolgt.

17. Ein Verfahren zur Herstellung von auf zumindest teilweise auf Methacrylsäurealkylestern basierenden Polymeren, beinhaltend die Schritte:
i. Herstellen eines Reinst-Methacrylsäurealkylesters nach einem Verfahren nach Anspruch 6, 7 oder 16;
ii. Polymerisieren des Reinst-Methacrylsäurealkylesters und gegebenenfalls ein Comonomer unter Erhalt eines Polymerisats;
iii. Aufarbeiten des Polymerisats unter Erhalt eines Polymers.

18. Verfahren nach Anspruch 17, wobei die Polymerisation durch radikalische Polymerisation erfolgt.

## Claims

1. Process for purifying an alkyl methacrylate to remove formic acid and/or methacrylic acid and/or sulphuric acid, comprising the steps of:
i. providing an alkyl methacrylate contaminated with formic acid and/or methacrylic acid and/or sulphuric acid;
ii. adsorbing at least some of the formic acid and/or methacrylic acid and/or sulphuric acid onto a purifying solid comprising at least 10% of a silicon-oxygen compound to obtain an ultrapure alkyl methacrylate.

2. Process according to Claim 1, wherein alkyl methacrylate has been distilled off for prepurification before step ii.

3. Process according to Claim 1 or 2, wherein the alkyl methacrylate provided in step i. includes at least one of the following impurities:
a. formic acid in an amount of not more than 0.05% by weight;
b. methacrylic acid in an amount of not more than 0.1% by weight;
c. sulphur dioxide in an amount of not more than 0.001% by weight.

4. Process according to one of the preceding claims, additionally comprising, as steps, the thermal regeneration of the purifying solid, followed by the reuse of the regenerated purifying solid in step ii.

5. Process according to one of the preceding claims, wherein at least step ii. is effected continuously.

6. Process for preparing an ultrapure alkyl methacrylate, comprising, as steps:
- producing an optionally prepurified alkyl methacrylate;
- purifying the alkyl methacrylate by a process according to one of the preceding claims.

7. Process according to Claim 6, wherein the production of the alkyl methacrylate, comprises the steps of
i. providing an acetone cyanohydrin;
ii. contacting the acetone cyanohydrin with an inorganic acid to obtain a methacrylamide;
iii. contacting the methacrylamide with an alcohol to obtain an alkyl methacrylate;
iv. optionally prepurifying the alkyl methacrylate.

8. Apparatus for preparing alkyl methacrylates, comprising, connected to one another in fluid-conducting form
- a plant element for preparing acetone cyanohydrin, followed by;
- a plant element for preparing methacrylamide, followed by;
- a plant element for preparing alkyl methacrylate, followed by;
- a plant element for purifying the alkyl methacrylate, followed by;
- a plant element for polymerization, followed by;
- a plant element for finishing, the plant element for purification comprising a vessel comprising a purifying solid comprising at least 10% of a silicon-oxygen compound.

9. Process according to one of Claims 1 to 7 or apparatus according to Claim 8, wherein a multitude of particles of the purifying solid are accommodated in a purifying vessel, and the purifying vessel can be flowed through by liquid alkyl methacrylate.

10. Process or apparatus according to one of the preceding claims, wherein the purifying solid has a specific surface area in a range of 400 to 1500 m²/g_{.}

11. Process or apparatus according to one of the preceding claims, wherein the purifying solid has a pore size, determined to DIN 66135-1, in the range of 0.01 to 2 nm.

12. Process or apparatus according to one of the preceding claims, wherein the purifying solid has a bulk density in the range of 920 to 960 mg/cm³.

13. Process or apparatus according to one of the preceding claims, wherein at least 80% by weight of the particles of the purifying solid have a particle size in the range of 0.5 to 10 mm.

14. Process or apparatus according to one of the preceding claims, wherein the weight-average particle size is in a range of 0.5 to 10 mm.

15. Process or apparatus according to one of the preceding claims, wherein the purifying solid includes at least 10% by weight of a zeolite.

16. Process according to Claim 6 or 7, which is effected in an apparatus according to one of Claims 8 to 15.

17. Process for preparing polymers based at least partly on alkyl methacrylates, comprising the steps of:
i. preparing an ultrapure alkyl methacrylate by a process according to Claim 6, 7 or 16;
ii. polymerizing the ultrapure alkyl methacrylate and optionally a comonomer to obtain a polymerization product;
iii. working up the polymerization product to obtain a polymer.

18. Process according to Claim 17, wherein the polymerization is effected by free-radical polymerization.

## Revendications

1. Procédé pour la purification d'un ester alkylique de l'acide méthacrylique, de l'acide formique et/ou de l'acide méthacrylique et/ou de l'acide sulfurique, comportant les étapes :
i. mise à disposition d'un ester alkylique de l'acide méthacrylique contaminé par de l'acide formique et/ou de l'acide méthacrylique et/ou de l'acide sulfurique ;
ii. adsorption d'au moins une partie de l'acide formique et/ou de l'acide méthacrylique et/ou de l'acide sulfurique sur un solide de purification contenant au moins 10% d'un composé de silicium-oxygène avec obtention d'un ester alkylique de l'acide méthacrylique pur.

2. Procédé selon la revendication 1, où l'ester alkylique de l'acide méthacrylique a été distillé avant l'étape ii. en vue d'une prépurification.

3. Procédé selon la revendication 1 ou 2, où l'ester alkylique de l'acide méthacrylique mis à disposition dans l'étape i. comporte au moins une des impuretés suivantes :
a. acide formique en une quantité d'au maximum 0,05% en poids ;
b. acide méthacrylique en une quantité d'au maximum 0,1% en poids ;
c. dioxyde de soufre en une quantité d'au maximum 0,001% en poids.

4. Procédé selon l'une quelconque des revendications précédentes, comportant en outre comme étapes la régénération thermique du solide de purification, suivie de la réutilisation du solide de purification régénéré dans l'étape ii.

5. Procédé selon au moins l'une quelconque des revendications précédentes, où au moins l'étape ii. a lieu de manière continue.

6. Procédé pour la préparation d'un ester alkylique de l'acide méthacrylique pur, comportant comme étapes
- production d'un ester alkylique de l'acide méthacrylique le cas échéant prépurifié ;
- purification de l'ester alkylique de l'acide méthacrylique par un procédé selon l'une quelconque des revendications précédentes.

7. Procédé selon la revendication 6, où la production de l'ester alkylique de l'acide méthacrylique comporte les étapes
i. mise à disposition d'une acétone-cyanhydrine ;
ii. mise en contact de l'acétone-cyanhydrine avec un acide inorganique avec obtention d'un méthacrylamide ;
iii. mise en contact du méthacrylamide avec un alcool avec obtention d'un ester alkylique de l'acide méthacrylique ;
iv. le cas échéant prépurification de l'ester alkylique de l'acide méthacrylique.

8. Dispositif pour la préparation d'esters alkyliques de l'acide méthacrylique, comportant, en étant reliés par voie fluidique les uns avec les autres,
- un élément d'installation pour la préparation d'acétone-cyanhydrine, suivi
- d'un élément d'installation pour la préparation de méthacrylamide, suivi
- d'un élément d'installation pour la préparation d'un ester alkylique de l'acide méthacrylique, suivi
- d'un élément d'installation pour la purification de l'ester alkylique de l'acide méthacrylique, suivi
- d'un élément d'installation pour la polymérisation, suivi
- d'un élément d'installation pour la confection,
où l'élément d'installation pour la purification comporte un récipient avec un solide de purification contenant au moins 10% d'un composé de silicium-oxygène.

9. Procédé selon l'une quelconque des revendications 1 à 7 ou dispositif selon la revendication 8, où une multitude de particules du solide de purification sont reprises dans un récipient de purification, le récipient de purification pouvant être traversé par un flux d'ester alkylique de l'acide méthacrylique liquide.

10. Procédé ou dispositif selon l'une quelconque des revendications précédentes, où le solide de purification présente une surface spécifique dans une plage de 400 à 1500 m²/g.

11. Procédé ou dispositif selon l'une quelconque des revendications précédentes, où le solide de purification présente une taille des pores déterminée par la norme DIN 66135-1 dans la plage de 0,01 à 2 nm.

12. Procédé ou dispositif selon l'une quelconque des revendications précédentes, où le solide de purification présente une densité apparente dans la plage de 920 à 960 mg/cm³.

13. Procédé ou dispositif selon l'une quelconque des revendications précédentes, où au moins 80% en poids des particules du solide de purification présentent une granulométrie dans la plage de 0,5 à 10 mm.

14. Procédé ou dispositif selon l'une quelconque des revendications précédentes, où la moyenne pondérale des particules se situe dans une plage de 0,5 à 10 mm.

15. Procédé ou dispositif selon l'une quelconque des revendications précédentes, où le solide de purification comporte au moins 10% en poids d'une zéolithe.

16. Procédé selon la revendication 6 ou 7, où le procédé est réalisé dans un dispositif selon l'une quelconque des revendications 8 à 15.

17. Procédé pour la préparation de polymères au moins partiellement à base d'esters alkyliques de l'acide méthacrylique, comportant les étapes :
i. préparation d'un ester alkylique de l'acide méthacrylique pur selon un procédé selon la revendication 6, 7 ou 16 ;
ii. polymérisation de l'ester alkylique de l'acide méthacrylique pur et le cas échéant d'un comonomère avec obtention d'un produit polymérisé ;
iii. traitement du produit polymérisé avec obtention d'un polymère.

18. Procédé selon la revendication 17, la polymérisation ayant lieu par voie radicalaire.
